# EUROPEAN PATENT APPLICATION

(11) **EP 4 810 108 A1**
(43) Date of publication of application: **23.09.2026**
(21) Application number: 24875741.1
(22) Date of filing: 16.10.2024
(51) Int. Cl.: A61N 5/06

(54) **PHOTOTHERAPY DEVICE FOR INHIBITING OR PREVENTING ALZHEIMER'S DISEASE**

(30) Priority: 15.11.2023 CN 202323093294 U
(71) Applicant: Danyang Huichuang Medical Equipment Co., Ltd., Zhenjiang, Jiangsu 212300 (CN)
(72) Inventor: WANG, Daifa, Zhenjiang, Jiangsu 212300 (CN); GUO, Genmiao, Zhenjiang, Jiangsu 212300 (CN); ZHAO, Xiaojing, Zhenjiang, Jiangsu 212300 (CN)
(74) Representative: Ziebig Hengelhaupt Intellectual Property Attorneys Patentanwaltskanzlei PartGmbB
(86) International application number: PCT/CN2024/125355
(87) International publication number: WO 2025/087131

(57) **Abstract**

Provided in the present application is a phototherapy device for inhibiting or preventing Alzheimer's disease. The phototherapy device comprises a support mechanism and an array of near-infrared irradiation units. The support mechanism is configured to form an accommodating space for a subject's head and to support the array of near-infrared irradiation units. The array of near-infrared irradiation units is configured to emit near-infrared light into the accommodating space and form, when the subject's head is positioned in place, an irradiation spatial range that covers at least the anterosuperior cranial region, the cranial vertex region, together with the left lateral cranial region and the right lateral cranial region. The time-averaged total radiant power incident upon the surface of the calvarial region of the subject's head is at least 27.5 W, or at least 31 W, or at least 40 W, or at least 60 W, or at least 70 W, and the spatiotemporally averaged irradiance over each covered region does not exceed 230 mW/cm². The phototherapy device does not rely on nasal or ocular irradiation, achieves optimized therapeutic effects for AD patients from MCI to dementia during treatment courses, and maintains and even continuously enhances therapeutic effects during phototherapy interruptions.

## Description

### TECHNICAL FIELD

The present application relates to a technical field of phototherapy devices for cerebral and cognitive diseases, and in particular relates to a phototherapy device for inhibiting or preventing Alzheimer's disease.

### BACKGROUND

Alzheimer's disease (AD) is a chronic, progressive neurodegenerative disease that mainly affects the elderly, especially those over 60 years of age. The disease is characterized by memory loss, loss of social and occupational function, impaired executive function, speech and motion deficits, personality changes, and behavioral and psychological disorders.

According to international guideline clinical staging, the course of AD can be divided into three stages: preclinical stage, mild cognitive disorders (MCI) stage, and dementia stage. The dementia stage can be further subdivided into mild, moderate, and severe dementia stages.

In the preclinical stage, biological samples collected from the individual's brain show changes in biomarkers that can be detected by medical means, such as abnormal changes in amyloid and tau proteins that can be identified by PET or biochemical testing of cerebrospinal fluid/blood, but no symptoms such as memory loss are manifested.

It should be noted that AD pathological changes, such as amyloid plaques (Aβ) in the cerebral cortex, may have already occurred many years before an AD diagnosis, even in the absence of any AD symptoms. It is currently hypothesized that such pathological changes require synergistic action with other pathological changes, such as neurofibrillary tangles (i.e., tau-PET positivity), to progress to the next pathological stage-the MCI stage. In the MCI stage, changes in AD biomarkers are accompanied by cognitive function changes, such as slow and mild deterioration of memory, language, and thinking functions.

Patients all will experience the MCI stage before entering the dementia stage. 15% of MCI cases will enter dementia stage after 2 years; approximately one-third will progress to dementia stage within 5 years; and about 26% of MCI cases will revert to normal cognition.

Upon entering the dementia stage, patients exhibit significant abnormalities in memory, language, thinking, and behavior that impair daily living activities, accompanied by marked abnormalities in AD-related biomarkers.

In recent years, photobiomodulation (PBM) has been introduced to treat AD. PBM involves delivering red or near-infrared light with a wavelength range of 600 to 1100 nm to the head, which penetrates the scalp and skull to act on brain tissue and treats AD non-invasively through multiple mechanisms such as increasing Aβ clearance, reducing abnormal aggregation of tau proteins, improving metabolism and mitochondrial function, increasing cerebral blood flow, and enhancing anti-oxidative stress and anti-inflammatory capabilities.

Photobiomodulation devices (also referred to as phototherapy devices herein) from different manufacturers vary greatly in the irradiation region over subjects, and problems also exist in practical application. For example, many manufacturers' phototherapy devices rely on near-infrared light irradiation incident upon the eyes of the subjects (see Liang Chen, etc., A Pilot Study of Near-Infrared Light Treatment for Alzheimer's Disease, Journal of Alzheimer's Disease 91 (2023) 191-201) or via nasal cavity of the subjects (see Anita E. Saltmarche, etc., Significant Improvement in Cognition in Mild to Moderately Severe Dementia Cases Treated with Transcranial Plus Intranasal Photobiomodulation: Case Series Report, Photomedicine and Laser Surgery, Volume 35, page 432-441) to irradiate the olfactory bulb. That is, a considerable part of the therapeutic efficacy of these devices is attributed to the dosage of near-infrared light that is irradiated to the olfactory bulb via the eyes or nose and further transmitted to the frontal lobe. However, AD patients are mostly elderly. During investigations and clinical trials in nursing homes, the inventors found that most elderly people show strong resistance to ocular and nasal irradiation, making implementation difficult. Moreover, ocular irradiation carries uncontrollable risks and injuries, including but not limited to infrared cataracts, retinal and choroidal damage, photosensitive cell damage, and corneal damage.

In addition, some phototherapy devices typically irradiate near-infrared light targeting the default mode network (DMN), or concentrate irradiation modules on the occipital lobe region without irradiating the parietal lobe. Irradiation regions vary and remain inconclusive.

Currently, regarding the clinical effects of phototherapy devices, attention is usually paid only to whether ADAS-cog scores decrease while MMSE scores increase after treatment, at most the magnitude of such score changes. Details of the change process-such as stagnation of score improvement during phototherapy, rapid deterioration and rebound of patient's cognitive status after phototherapy cessation, and learning effects due to excessively short follow-up intervals-are overlooked.

Accordingly, research on the action mechanism of existing phototherapy devices during and after treatment is relatively crude. There is ample room for improvement to achieve optimally sustained therapeutic effects in a broader AD population of treatment subjects (from MCI to severe dementia, including those who cannot accept nasal or ocular irradiation).

### SUMMARY

In view of the above problems in the prior art, an object of the present application is to provide a phototherapy device for inhibiting or preventing Alzheimer's disease that does not rely on nasal or ocular irradiation, achieves optimized therapeutic effects for AD patients from MCI to dementia during treatment courses, and maintains and even continuously enhances therapeutic effects during phototherapy interruptions.

According to a first aspect of the present application, a phototherapy device for inhibiting or preventing Alzheimer's disease is provided. The phototherapy device comprises a support mechanism and an array of near-infrared irradiation units. The support mechanism is configured to form an accommodating space for a subject's head and to support the array of near-infrared irradiation units. The array of near-infrared irradiation units is configured to emit near-infrared light into the accommodating space and form, when the subject's head is positioned in place in the accommodating space, an irradiation spatial range that covers at least the anterosuperior cranial region, the cranial vertex region, together with the left lateral cranial region and the right lateral cranial region on a surface of a calvarial region of the subject's head. Wherein, the time-averaged total radiant power incident upon the surface of the calvarial region of the subject's head is at least 27.5 W, or at least 31 W, or at least 40 W, or at least 60 W, or at least 70 W, and the spatiotemporally averaged irradiance over each covered region does not exceed 230 mW/cm².

Compared with the prior art, the beneficial effects of the embodiments of the present application are as follows. The phototherapy device is not limited to a specific structure, nor is it limited to whether the near-infrared irradiation units are ordinary LEDs or low-energy laser diodes. By means of the phototherapy device, near-infrared light with sufficient total power are incident upon at least the anterosuperior cranial region, the cranial vertex region, together with the left lateral cranial region and the right lateral cranial region on the surface of the calvarial region of the subject's head, avoiding nasal and transcranial irradiation that may cause discomfort or resistance in some patients. This irradiation spatial range ensures that the irradication of near-infrared light can modulate core network nodes and brain regions of the default mode network (DMN), central executive network (ECN, also known as executive control network), salience network (SN), sensorimotor network (SMN), and dorsal attention network (DAN). Applying a time-averaged total radiant power of at least 27.5 W onto the above regions of the surface of the calvarial region of the subject's head altogether can effectively inhibit or interrupt the progression of neurodegenerative lesions caused by abnormal Aβ plaques and tau proteins from spreading along brain functional networks. In clinical trials, the phototherapy device has been confirmed to exert certain therapeutic effects on multiple individual AD patients across multiple disease stages ranging from MCI to dementia. Furthermore, clinical trials on approximately 27 initial patients with mild to moderate dementia (including an intervention group and a sham control group) have validated that the therapeutic effect of the phototherapy device increases steadily during treatment courses, and the therapeutic effect is maintained for a certain interruption period after each course. Even the biochemical reactions triggered by previous course(s) continue to induce inhibitory effects, and the patients exhibited continued clinical improvement. Although the exact underlying mechanism has not been fully elucidated, the phototherapy device of the present application indeed exhibits excellent "optical-charging depth" and "runtime per charge" in inhibiting AD progression, avoiding deterioration and rebound during interruptions, continuously increasing benefits, and causing no adverse reactions in patients.

It should be understood that the foregoing general description and the following detailed description are exemplary and explanatory only and are not restrictive of the present invention.

The overview of various implementations or examples of the technology described in this application is not a comprehensive disclosure of the full scope or all features of the disclosed technology.

### BRIEF DESCRIPTION OF THE DRAWINGS

In figures that are not necessarily drawn to scale, the same reference numerals may describe similar components in different figures. The same reference signs with letter suffixes or different letter suffixes may denote different examples of similar components. The figures generally show various embodiments by way of example rather than limitation, and are used together with the description and the claims to describe the embodiments of the present invention. When appropriate, the same reference sign may be used throughout the drawings to denote the same or similar part. Such embodiments are illustrative, and are not intended to be exhaustive or exclusive embodiments of the present device or method.
FIG. 1 is a longitudinal sectional structural schematic view of a headgear of a phototherapy device according to a first embodiment of the present application.
FIG. 2 is a longitudinal sectional structural schematic view of a headgear of a phototherapy device according to a second embodiment of the present application.
FIG. 3 is a bottom structural schematic view of the headgear of the phototherapy device according to the first or second embodiment of the present application.
FIGS. 4(a)-4(f) show diagrams of characteristic parameters of the head of a treatment subject, or a reference head phantom of a population of treatment subjects according to a third embodiment of the present application.
FIG. 5(a) shows a front view of a reference head phantom as an example of a subject's head according to a fourth embodiment of the present application, where electrode positions of the 10-10 standard system, a total boundary line of a reference calvarial region, and boundary lines among the anterosuperior cranial region, the left lateral cranial region, and the right lateral cranial region are illustrated on the subject's head.
FIG. 5(b) shows a left side view of the reference head phantom as an example of a subject's head according to the fourth embodiment of the present application, where electrode positions of the 10-10 standard system, the total boundary line of the reference calvarial region, and boundary lines among the anterosuperior cranial region, the left lateral cranial region, the cranial vertex region, and the posterior cranial region are illustrated on the subject's head.
FIG. 5(c) shows a right side view of the reference head phantom as an example of a subject's head according to the fourth embodiment of the present application, where electrode positions of the 10-10 standard system, the total boundary line of the reference calvarial region, and boundary lines among the anterosuperior cranial region, the right lateral cranial region, the cranial vertex region, and the posterior cranial region are illustrated on the subject's head.
FIG. 5(d) shows a top view of the reference head phantom as an example of a subject's head according to the fourth embodiment of the present application, where electrode positions of the 10-10 standard system, the total boundary line of the reference calvarial region, and boundary lines among the anterosuperior cranial region, the cranial vertex region, the left lateral cranial region, the right lateral cranial region, and the posterior cranial region are illustrated on the subject's head.
FIG. 5(e) shows a rear view of the reference head phantom as an example of a subject's head according to the fourth embodiment of the present application, where electrode positions of the 10-10 standard system, the total boundary line of the reference calvarial region, and boundary lines among the cranial vertex region, the left lateral cranial region, the right lateral cranial region, and the posterior cranial region are illustrated on the subject's head.
FIG. 5(f) shows an exemplary diagram of the irradiation regions according to a fifth embodiment of the present application.
FIG. 6(a) shows a schematic diagram of electrode positions on the anterosuperior cranial region of a reference head phantom as an example of a subject's head based on the 10-10 standard system according to a sixth embodiment of the present application.
FIG. 6(b) shows a schematic diagram of electrode positions on the anterosuperior cranial region of a reference head phantom as an example of a subject's head based on the 10-10 standard system according to a seventh embodiment of the present application.
FIG. 6(c) shows a schematic diagram of electrode positions on the anterosuperior cranial region of a reference head phantom as an example of a subject's head based on the 10-10 standard system according to an eighth embodiment of the present application.
FIGS. 7(a)-7(c) show division manners of the anterosuperior cranial region, cranial vertex region, the left lateral cranial region, and the right lateral cranial region according to three embodiments of the present application.
FIG. 8 shows a schematic diagram of spatial positions of the default mode network (DMN), central executive network (ECN, also known as executive control network), salience network (SN), sensorimotor network (SMN), dorsal attention network (DAN), and visual network (VN) in the brain.
FIG. 9 shows a schematic diagram of an arrangement structure of a irradiation panel according to the eighth embodiment of the present application.
FIG. 10 shows a schematic diagram of an arrangement structure of a irradiation panel frame according to a ninth embodiment of the present application.
FIG. 11 shows a schematic flowchart of a clinical trial using a phototherapy device according to an embodiment of the present application on AD patients.
FIG. 12(a) shows a trend graph of ADAS-Cog scores of a sham control group before near-infrared phototherapy, during near-infrared phototherapy, and after near-infrared phototherapy cessation.
FIG. 12(b) shows a trend graph of ADAS-Cog scores of an intervention group before near-infrared phototherapy, during near-infrared phototherapy, and after near-infrared phototherapy cessation.
FIG. 13(a) shows a trend graph of MMSE scores of a sham control group before near-infrared phototherapy, during near-infrared phototherapy, and after near-infrared phototherapy cessation.
FIG. 13(b) shows a trend graph of MMSE scores of an intervention group before near-infrared phototherapy, during near-infrared phototherapy, and after near-infrared phototherapy cessation.

### Reference Signs

1-outer housing; 2-middle housing; 3-inner housing; 4-irradiation panel accommodating cavity; 5-irradiation panel; 6-cold air cavity; 7-ventilation hole; 701-upper layer of ventilation hole units; 702-middle layer of ventilation hole units; 703-lower layer of ventilation hole units; 8-cold air inlet; 9-irradiation panel fixing housing; 10-first layer of irradiation panels; 11-second layer of irradiation panels; 12-third layer of irradiation panels; 13-fourth layer of irradiation panels; 14-fifth layer of irradiation panels; 15-sixth layer of irradiation panels; 16-vent opening; 17-irradiation panel fixing frame; 18-connecting part.

### DETAILED DESCRIPTION

To make the objectives, technical solutions, and advantages of the embodiments of the present application clearer, the technical solutions in the embodiments of the present application will be clearly and completely described below in conjunction with the accompanying drawings of the embodiments of the present application. Obviously, the described embodiments are part and non-exhaustive of the embodiments of the present application. All other embodiments obtained by persons of ordinary skill in the art without creative work based on the described embodiments of the present application shall fall within the protection scope of the present application.

Technical or scientific terms used in the present application shall have the ordinary meaning as understood by persons of ordinary skill in the art to which the present application belongs, unless otherwise defined. The terms "first", "second" and similar words used in the present application do not indicate any sequence, quantity or importance, but are only used for distinguishing different parts. Words like "comprising" or "including" and similar words mean that the elements or object preceding the word cover the elements or object and their equivalent listed after the word, and do not exclude other elements or objects. Similar words such as "connected" or "connected with" are not limited to physical or mechanical connection, but can also include electrical connection, whether direct or indirect. The terms "upper", "lower", "left", "right", etc. are only used to represent relative positional relationships. When the absolute position of the described object changes, the relative positional relationship may also change accordingly.

The so-called "inhibiting or preventing Alzheimer's disease" in the present application is intended to mean exerting effects of early prevention, probability reduction, alleviation, inhibition, termination, or even reversal on the progression of the course of AD. The course of AD is intended to include not only the process in which a subject has developed clinical symptoms of AD and subsequent progression, but also the process in which the subject has not yet developed obvious clinical symptoms of AD but some pathological or physiological phenomena associated with AD and likely to progress to AD with a certain probability have occurred. That is, the expression "inhibiting or preventing Alzheimer's disease" in the present application includes treating existing Alzheimer's disease and also includes preventing the occurrence of Alzheimer's disease or reducing the probability of developing Alzheimer's disease. Specifically, according to the 2018 AD diagnostic criteria of the National Institute on Aging and Alzheimer's Association (NIA-AA), biomarkers can be classified into four categories and cognitive function can be further graded into six levels based on examinations of β-amyloid (Aβ) and tau in the brain or cerebrospinal fluid of the population, as well as cranial MRI and FDG-PET. Level 1: normal objective neuropsychological tests, no cognitive complaints, no neurobehavioral symptoms, no informant-reported cognitive decline or neurobehavioral symptoms, no follow-up test evidence of cognitive decline. Level 2: subjective cognitive decline (SCD), objective slight cognitive decline (Obj-SCD), and neurobehavioral symptoms. Levels 1 and 2 are collectively referred to as the preclinical stage. Level 3: abnormal or impaired objective cognitive tests but not meeting dementia criteria, i.e., MCI. Levels 4-6: mild, moderate, and severe dementia, respectively. Each stage among these six levels with positive biomarker test results falls within the course of AD as defined in the present application. Furthermore, for subjects carrying genes associated with AD risk (such as APOE ε4, ABCA7, CLU, CR1, PICALM, PLD3, and TREM2) but currently testing negative for Aβ, , even if cognitive function is at Level 1, the administration of medical intervention to reduce their risk of developing AD and slow down the progression to AD may also be regarded as "inhibiting or preventing Alzheimer's disease" in the present application.

The so-called "calvarial region of the subject's head" in the present application is intended to cover "calvarial region" as defined in various ways in anthropological and medical fields, unless specifically limited. For example, the "calvarial region" may adopt the boundary used by Friess et al. (2002) when measuring calvarial area. As another example, a plane formed by the supraglabella point and superior margin points of both external auditory canals may be used as a boundary plane, and the part of the head above this boundary plane is taken as the "calvarial region". As another example, the "calvarial region" may also refer to the part on the surface of the head surrounded by a total boundary line, which can be defined according to the 10-10 standard system (i.e., the international 10/10 system for EEG electrodes) as that passing from a glabella point of the subject's head through the preauricular points on both sides, and extending posteriorly around the head, passing between the inion and the electrode positions O1, OZ, and O2 of the 10-10 standard system to converge.

The so-called "incident upon the surface of calvarial region of the subject's head" in the present application is intended to mean the irradiation is delivered to (i.e., lands on) the outer thin-layer irradiation surface adjacent to the hair (or scalp where there is no hair) of the calvarial region of the subject's head (i.e., the irradiation lands on the calvarial region of the subject's head). Irradiation of near-infrared light landing on this outer thin-layer irradiation surface means that the irradiation energy of the near-infrared light is delivered to the calvarial region including hair, scalp, skull, and then delivered to the brain tissue. Furthermore, after the delivered energy is absorbed by hair and attenuation by the scalp and skull, the remaining energy is capable of arriving at (acting on) the cerebral cortex and even deeper parts of the brain tissue, and the remaining energy is correlated with the attenuation occurring along the transmission path.

The so-called "time-averaged total radiant power" in the present application is distinguished from peak radiant power and is intended to mean the total radiant power level obtained by time averaging the total irradiation energy accumulated over a period of time.

In the present application, the so-called "time-averaged irradiance" is intended to mean the irradiance averaged over time. For example, the "time-averaged irradiance" at a target point (i.e., position) is intended to mean the irradiance averaged over time at the target point. As another example, the "time-averaged irradiance" over a target region is intended to mean the "time-averaged irradiance" at representative positions on the target region. Specifically, a "time-averaged irradiance" of 30-60 mW/cm² over a target region means that the time-averaged irradiance at each representative position on the target region (such as but not limited to the position corresponding to the center of a irradiation panel) fluctuates within the range of 30-60 mW/cm².

The so-called "spatiotemporally averaged irradiance" over a target region is intended to mean a parameter obtained by performing an averaging operation over the surface of the target region (over its surface area) based on the time-averaged irradiances (at the representative positions on the target region), that is, the optical irradiance obtained by performing averaging operations over both the surface area (spatio-) and time (temporally).

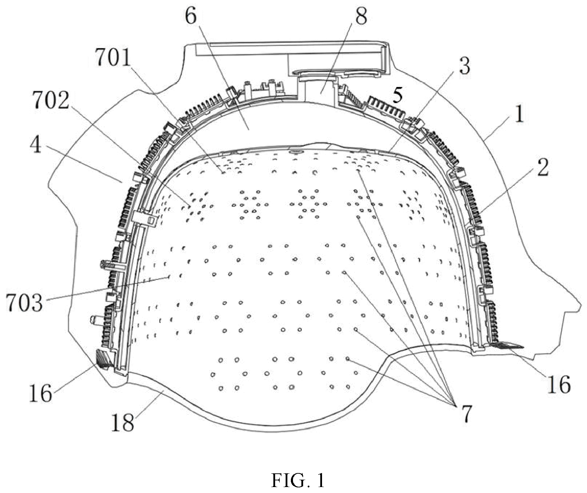

According to a first aspect of the present application, a phototherapy device for inhibiting or preventing Alzheimer's disease is provided. The phototherapy device may comprise a support mechanism, such as but not limited to the headgear shown in FIGS. 1-3, configured to form an accommodating space for a subject's head and to support an array 5 of near-infrared irradiation units. In some embodiments, the support mechanism may adopt a loose headgear as shown in FIGS. 1-3, such that when the head of an AD patient is positioned in place therein, an appropriate distance of several centimeters is maintained between the head and the inner wall of the headgear, providing sufficient clearance for head movement of the AD patient without causing worry or anxiety of the AD patient due to excessive space. Note that the so-called "the subject's head is positioned in place in the accommodating space" herein is intended to mean that the subject's head is set up in a standard treatment position or within an allowable position deviation range from the standard treatment position in the accommodating space. For example, in the standard treatment position, the center of the subject's head may be aligned with the center of the accommodating space, and the front-rear median axes are aligned with each other. As another example, in the standard treatment position, the subject's head may be centered in the accommodating space, with substantially consistent front and rear distances from the front and rear walls of the accommodating space and substantially consistent left and right distances from the left and right walls of the accommodating space. As another example, taking the phototherapy headgear shown in FIGS. 1-3 as an example, when positioned in place, the brow ridge of the subject's head is flush with the front edge of the headgear, and the subject's head is centered in the headgear, such that the front and rear distances from the irradiation surface of the inner housing are substantially consistent and the left and right distances from the irradiation surface of the inner housing are also substantially consistent.

In some embodiments, the support mechanism may also be implemented as a skeleton structure closely attached to the head of an AD patient, such that the near-infrared irradiation units or sub-arrays thereof can be mounted on the skeleton structure as modules. In still other embodiments, the support mechanism may also be implemented as a non-headgear support frame located farther from the head of an AD patient, adopting various structures such as an arc shape, a cone shape, or a spatial grid shape, as long as it supports the array 5 of near-infrared irradiation units, which will not be repeated herein.

The array 5 of near-infrared irradiation units is specifically configured to emit near-infrared light into the accommodating space, forming, when the subject's head is positioned in place in the accommodating space, an irradiation spatial range that covers at least the anterosuperior cranial region, the cranial vertex region, together with the left lateral cranial region and the right lateral cranial region on the surface of a calvarial region of the subject's head. The time-averaged total radiant power incident upon (acting on) the surface of calvarial region of the subject's head is at least 27.5 W, and the spatiotemporally averaged irradiance over each covered region does not exceed 230 mW/cm², to avoid the risk of thermal damage.

In some embodiments, a controller and a driving circuit may be provided in a host separated from the headgear and wired to the near-infrared irradiation units to control and drive each near-infrared irradiation unit via wiring so as to provide the required time-averaged total radiant power and spatiotemporally averaged irradiance. As another example, a controller and a driving circuit may also be provided in the headgear to control and drive each near-infrared irradiation unit so as to provide the required time-averaged total radiant power and spatiotemporally averaged irradiance.

Specifically, the anterosuperior cranial region on the surface of the calvarial region is mainly associated with the frontal lobe, the cranial vertex region on the surface of the calvarial region is mainly associated with the parietal lobe, and the left lateral cranial region and the right lateral cranial region on the surface of the calvarial region are mainly associated with the left and right temporal lobes. It should be understood that this association implies not only spatial correlation but also connectivity correlation in functional brain network. That is, if sufficient irradiation of near-infrared light is delivered to the anterosuperior cranial region, it also enables neuromodulation of the frontal lobe, and the same applies to other regions. The inventors have found that delivering a time-averaged total radiant power of at least 27.5 W directly to (the surface of) the calvarial region of the subject's head, with an irradiation spatial range covering at least the anterosuperior cranial region, the cranial vertex region, the left lateral cranial region, and the right lateral cranial region, can deliver irradiation of near-infrared light with sufficient energy to core network nodes and brain regions of the default mode network (DMN), central executive network (ECN, also known as executive control network), salience network (SN), sensorimotor network (SMN), and dorsal attention network (DAN), thereby effectively inhibiting or interrupting the progression of neurodegenerative lesions caused by abnormal Aβ plaques and tau proteins from spreading along brain functional networks. The inventors have creatively discovered that focusing solely on the average irradiance (i.e., radiant power level per unit area, mW/cm²) or solely on the irradiation energy "charged" per unit area (J/cm², e.g., radiant fluence per session) cannot ensure robust therapeutic effects against global brain lesions in AD. Specifically, AD is a global brain disease in which lesions spread from some regions along functionally connected regions of brain functional networks rather than along spatially related but poorly connected sites. Furthermore, AD lesions induce specific responses in specific cell populations, such as but not limited to Astrocytes, Microglia, Oligodendrocytes, Neurons, Vascular Cells, Peripheral Glial Cells, and Extracellular Matrix. By applying sufficient time-averaged total radiant power to cell populations, it not only generates a change response to inhibit AD itself, but also can spread the change response along the functionally connected regions and deliver it to other cell populations. Note that the positioning and coverage of the anterosuperior cranial region, the cranial vertex region, together with the left lateral cranial region and the right lateral cranial region in the present application exactly correspond to or target the core network nodes and brain regions of the above brain networks. By delivering sufficient time-averaged total radiant power to the core network nodes and brain regions of these networks, the spreading paths along functionally connected regions is smoother and the spreading and propagation effect of the inhibiting response is more efficient, thereby achieving optimized AD inhibition effects in the whole brain. If the process of delivering the irradiation of near-infrared light to the subject's head is analogized to battery charging, e.g., referred to as a "optical-charging" process, the phototherapy device according to the present application can achieve greater "optical-charging capacity", deeper "optical-charging depth", and faster "optical-charging speed" in the subject's brain, thus providing better subsequent "runtime per charge" (i.e., the duration for which therapeutic effects are maintained or even enhanced after optical charging). In addition to the total radiant power (incident upon the surface of the calvarial region of the subject's head) used in clinical trials on a subject population described below, the inventors also adopted multiple levels of total radiant power, such as at least 31 W , or at least 40 W, or at least 60 W, or at least 70 W, specifically various total radiant power values from 29 W to 135 W at 1 W intervals, such as these total radiant power levels of 30 W, 32 W, 34 W, 35 W, 38 W, 45 W, 50 W, 60 W, 65 W, 70 W, 75 W, 80 W, 85 W, 90 W, 95 W, 100 W, 105 W, 110 W, 115 W, 120 W, 125 W, 130 W, and 135 W, etc. Phototherapy effects were tested on individual AD patients, confirming that patients had no adverse reactions at total radiant powers within this range, and AD patients reported relief of previous cognitive disorders such as recent memory loss, muscle rigidity, and weakened spatial ability.

The subject's head comprises the head of a treatment subject, or a reference head phantom of a population of treatment subjects.

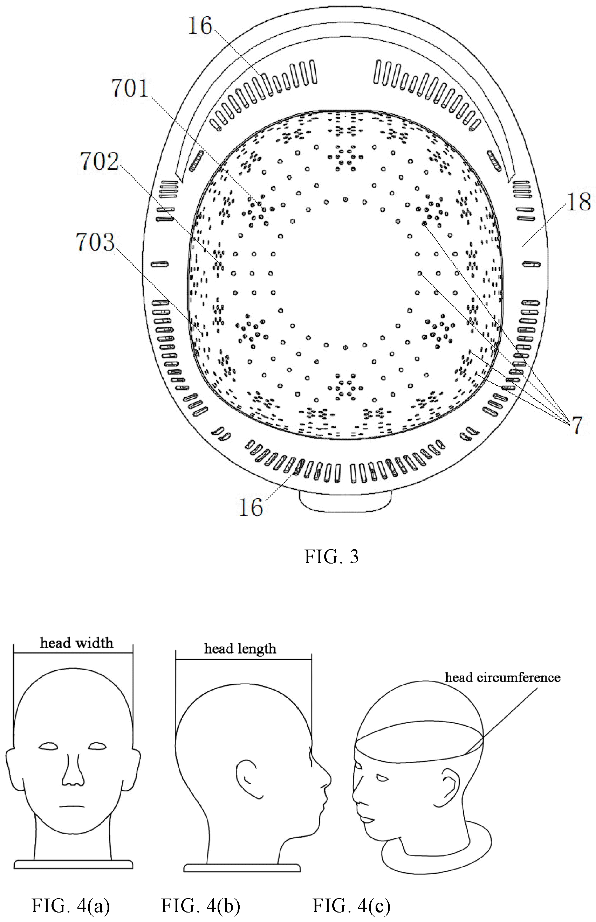

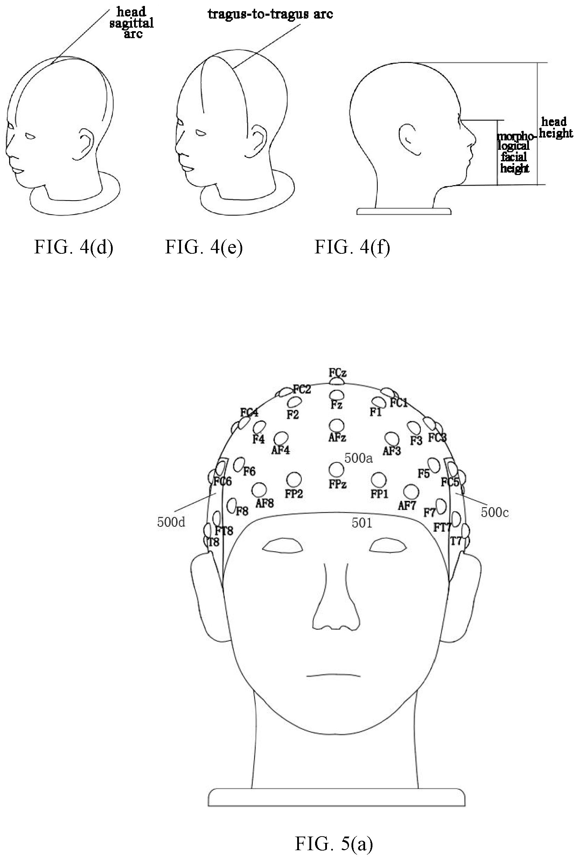

In some embodiments, during manufacturing of the phototherapy device, a reference head phantom of a population of treatment subjects may be used to simulate attenuation and transmission of near-infrared light. Specifically, a reference head phantom with representative dimensions may be selected according to a population of treatment subjects. For example, the concentrated age group of AD patients is over 60 years old. For a population of treatment subjects of this age group, parameters of the reference head phantom may be: a head width of 140-166 mm, a head length of 170-196 mm, a head circumference of 525-583 mm, a morphological facial height of 104-130 mm, a head sagittal arc of 304-372 mm, a tragus-to-tragus arc of 320-375 mm, and a head height of 206-253 mm, as shown in FIGS. 4(a)-4(f).

Specifically, the parameter range of the reference head phantom falls within the intersection of the distribution ranges of P1, P5, P10, P50, P90, P95, and P99 parameter values for females in this age group and P1, P10, P50, P90, P95, and P99 parameter values for males in this age group, thus having good representativeness for both males and females in this age group.

In some embodiments, parameters of the reference head phantom may be refined as: a head width of 152 mm, a head length of 184 mm, a head circumference of 536.7 mm, a morphological facial height of 109.3 mm, a head sagittal arc of 355.6 mm, a tragus-to-tragus arc of 324.1 mm, head height of 206 mm. At least part of these refined parameters is determined based on P50 parameter values for females in this age group and P50 parameter values for males in this age group. For example, the head width and head length herein are each averages of corresponding two P50 parameter values. P50 parameter values for females in this age group are as follows: a head width of 149 mm, a head length of 180 mm, a head circumference of 548 mm, a morphological facial height of 111 mm, a head sagittal arc of 335 mm, a tragus-to-tragus arc of 342 mm, and a head height of 228 mm. P50 parameter values for males in this age group are as follows: a head width of 155 mm, a head length of 188 mm, a head circumference of 565 mm, a morphological facial height of 121 mm, a head sagittal arc of 343 mm, a tragus-to-tragus arc of 351 mm, and a head height of 231 mm. It can be seen that the parameters of the reference head phantom are highly consistent with the P50 parameters for females and males in this age group, thus being more representative. Furthermore, the cephalo-facial index of the reference head phantom with these parameters is 82%, which also conforms to the cephalo-facial index range of the dominant head type- Brachycephaly in Chinese (and a population of East Asian). Therefore, the parameters of the reference head phantom are particularly representative in Chinese and a population of East Asian. In some embodiments, for populations with other cephalo-facial indices, such as but not limited to major population of Europe, South Asia, and Africa, each parameter may be adaptively adjusted for good representativeness.

The phototherapy device of the present application has been tested on multiple reference head phantoms (such as but not limited to those representing elderly East Asian males and females, elderly African-American males and females, etc.), confirming that when each reference head phantom is positioned in place in the accommodating space, at least the anterosuperior cranial region, the cranial vertex region, together with the left lateral cranial region and the right lateral cranial region on the surface of its calvarial region can be covered and the surface of its calvarial region can receive sufficient time-averaged total radiant power.

In some embodiments, during manufacturing of the phototherapy device, a representative reference head phantom may be prepared for a target population of subject, and verification of the irradiation spatial range may be performed on the surface of the calvarial region of the reference head phantom. Verification of the irradiation spatial range on the surface of the calvarial region may then be performed for a target population of subject within a predetermined range, so as to ensure that when the head of an individual in the target population of subject is positioned in place in the accommodating space, at least the anterosuperior cranial region, the cranial vertex region, together with the left lateral cranial region and the right lateral cranial region on the surface of the calvarial region of its head are sufficiently covered and the surface of the calvarial region of its head receive sufficient time-averaged total radiant power.

In a fourth embodiment of the present application, the calvarial region, the anterosuperior cranial region, the cranial vertex region, together with the left lateral cranial region and the right lateral cranial region are all defined on the subject's head based on electrode positions of the 10-10 standard system, as shown in FIGS. 5(a)-5(e). In FIGS. 5(a)-5(f), protrusions are drawn at each electrode position for clarity; however, in some embodiments, protrusions may be not provided on the surface of the reference head phantom. The 10-10 standard system is an electrode placement standard for electroencephalogram (EEG) recording, providing an accurate and consistent method for marking and positioning electrodes on the head. That is, the 10-10 system can be directly applied to the subject's head without transcranial procedures. This system is an extension of the earlier 10-20 system proposed by the International Federation of Clinical Neurophysiology to standardize electrode positions in EEG recording.

In the 10-10 system, electrode positions are located based on anatomical landmarks of the head, comprising the nasion, inion, and pre-auricular points. By means of these landmarks, the front-rear and left-right median lines of the head can be determined, and electrodes are placed at 10% intervals accordingly.

The naming convention of the 10-10 system is based on the 10-20 system but provides denser electrode placement, especially at the bottom and front of the temporal lobe and the frontal lobe, locating positions often overlooked in the 10-20 system. In addition, the 10-10 system introduces new electrodes to allow more precise positioning division at the junctions of brain regions, such as FC for representing electrodes between frontal and central regions, FT for representing electrodes between frontal and temporal regions, CP for representing electrodes between central and parietal regions, and PO for representing electrodes between parietal and occipital regions. Electrode placement in the 10-10 system can effectively eliminate the influence of differences in the shape and size of individual head, enabling the same electrode position to be substantially accurately located at the same brain region node in different individuals. Its denser electrode positions, especially electrode positions for the temporal and frontal lobes, enable finer division of the anterosuperior cranial region, the cranial vertex region, together with the left lateral cranial region and the right lateral cranial region on the surface of the calvarial region (outside the skull, scalp, and hair), and correspondingly finer division of brain regions (frontal lobe, parietal lobe, temporal lobe) under the skull and dura mater.

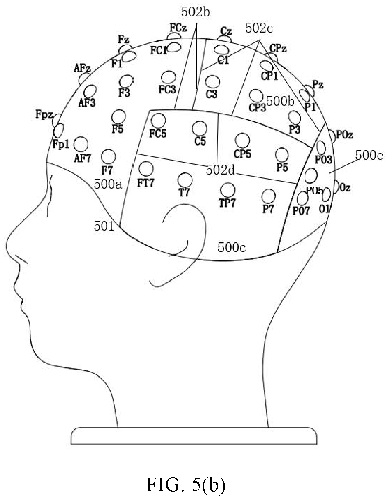

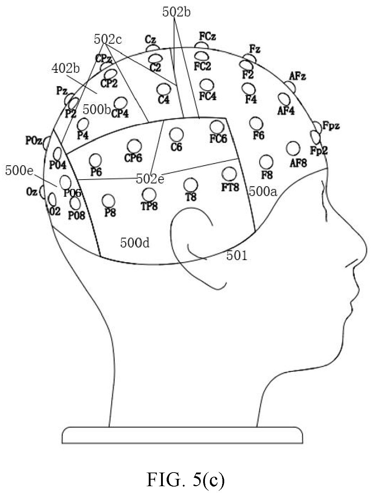

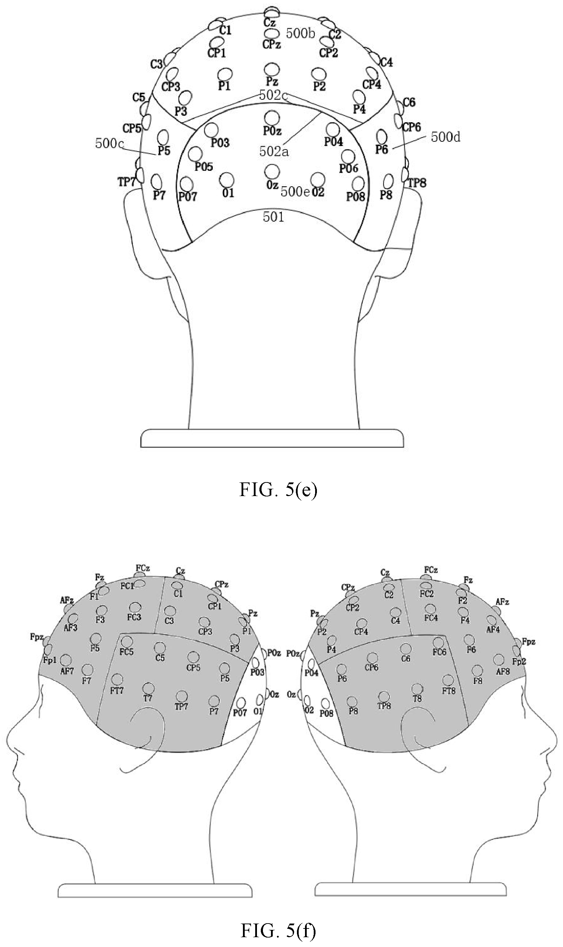

Referring to FIGS. 5(a)-5(e), the anterosuperior cranial region 500a, the cranial vertex region 500b, the left lateral cranial region 500c, and the right lateral cranial region 500d, together with posterior cranial region 500e are arranged in blocks and located within a total region enclosed by a total boundary line 501. The total boundary line 501 passes from the glabella point of the subject's head (see FIG. 5(a)) through preauricular points on both sides (see FIGS. 5(b) and 5(c)), and extends posteriorly around the head, passing between the inion and the electrode positions O1, OZ, and O2 of the 10-10 standard system to converge (see FIG. 5(e)). Note that for clarity of electrode positions, small protrusion and name of the electrode positions are marked together at each electrode position in the present application; however, the small protrusions may be absent.

The total boundary line 501 may be used to define the surface of the calvarial region, that is, the area of the total region enclosed by the total boundary line 501 is the total area of the surface of the calvarial region. Although in FIGS. 5(a)-5(e), the anterosuperior cranial region 500a, the cranial vertex region 500b, the left lateral cranial region 500c, and the right lateral cranial region 500d, together with posterior cranial region 500e occupy the entire total region, this is merely an example, and these regions may occupy only a part of the total region, e.g., at least 60%, or at least 70%, or at least 80% of its surface. As another example, the proportion of these regions occupying the surface of the total region may be any percentage in the range of 60% to 100% at 1% intervals.

In some embodiments, the anterosuperior cranial region 500a, the cranial vertex region 500b, together with the left lateral cranial region 500c and the right lateral cranial region 500d are located within a first region divided from the total region by a fifth boundary line 502a and occupy at least 60%, or at least 70%, or at least 80% of the surface area of the first region, as shown in FIG. 5(e). According to the 10-10 standard system, the fifth boundary line 502a sequentially passes between the following electrode positions (see FIGS. 5(b), 5(c), and 5(e)): between P7 and PO7, between P5 and PO5, between P3 and PO3, between P1 and POZ, between PZ and POZ, between P2 and POZ, between P4 and PO4, between P6 and PO6, and between P8 and PO8. The so-called "passing between electrode positions A and B" in the present application is intended to mean passing through an intermediate point on the line connecting electrode positions A and B. For example, the intermediate point may be the midpoint of the connecting line or another point on the connecting line, such as a point at a position ratio of 1:2 from electrode positions A and B. In some embodiments, for the same boundary line, e.g., the fifth boundary line 502a, the points passing between paired electrode positions may be at different ratios on the connecting lines to ensure that the sequentially connected boundary line is smooth. By means of delivering sufficient time-averaged optical (irradiation) power, e.g., at least 27.5 W, or at least 31 W, or at least 40 W, or at least 60 W, or at least 70 W, to at least 60%, or at least 70%, or at least 80% of the surface area of the first region, core network nodes and brain regions of the default mode network (DMN), central executive network (ECN, also called executive control network), salience network (SN), sensorimotion network (SMN), and dorsal attention network (DAN) can be substantially covered. Even with slight deviations, sufficient radiant power ensures that radiant power components at deviated nodes break through spatial intervals to spread to adjacent core network nodes and brain regions.

In addition, enabling the anterosuperior cranial region 500a, the cranial vertex region 500b, together with the left lateral cranial region 500c and right lateral cranial region 500d to jointly bear sufficient time-averaged radiant power not only eliminates dependence on ocular and nasal irradiation, but also makes the subject's treatment experience more comfortable and relaxed. For example, when using a light guide comb of the present inventors, dark hair is parted on the forehead, cranial vertex, and cranial sides to leave hair-free blank areas on the scalp, optimizing near-infrared light transmission, specifically improving light transmittance of dark thick hair and scalp by 20-30%. However, the parted and then gathered hair is combed backward and eventually superimposed on the occipital region. Once dependence on the occipital region (corresponding to the posterior cranial region 500e) is eliminated, as shown in the irradiation range of FIG. 5(f), dark hair in the anterosuperior cranial region 500a, the cranial vertex region 500b, together with the left lateral cranial region 500c and the right lateral cranial region 500d can be better parted to improve light transmittance. Furthermore, since the occipital region is at a lower position and has a complex curved and partially concave shape, near-infrared light from the surrounding area will be substantially blocked unless irradiation units are arranged contacting against the scalp. By means of irradiation tests using the phototherapy device shown in FIGS. 1-3 of the present application, it is validated that for modifying the near-infrared irradiation scheme, compared to increasing irradiance over the posterior cranial region 500e, it is more efficient and easier to increase irradiance over the anterosuperior cranial region 500a, the cranial vertex region 500b, together with the left lateral cranial region 500c and the right lateral cranial region 500d.

In some embodiments, the anterosuperior cranial region 500a, the cranial vertex region 500b, together with the left lateral cranial region 500c and the right lateral cranial region 500d may be divided as follows, so as to correspond and cover more precisely the core network nodes and brain regions of the default mode network (DMN), central executive network (ECN), salience network (SN), sensorimotion network (SMN), and dorsal attention network (DAN).

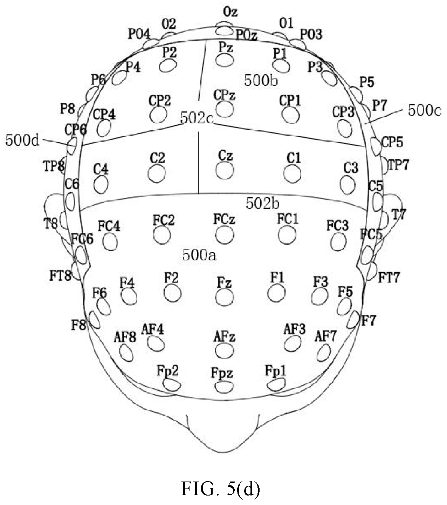

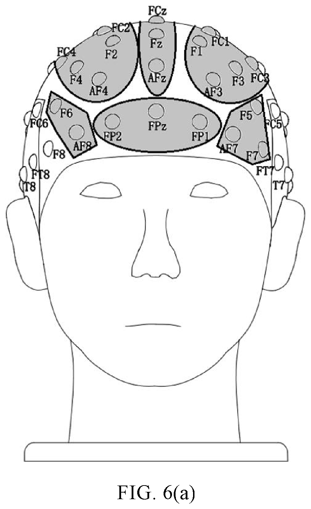

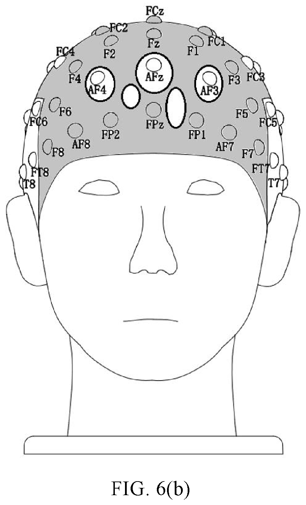

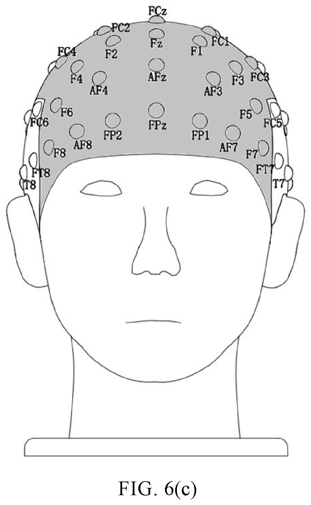

In some embodiments, the anterosuperior cranial region 500a, the cranial vertex region 500b, together with the left lateral cranial region 500c and the right lateral cranial region 500d may be divided according to their boundary lines. Specifically, the anterosuperior cranial region 500a is located in a first sub-region enclosed by a first boundary line 502b and the total boundary line 501. According to the 10-10 standard system, the first boundary line 502b sequentially passes between the following electrode positions: between F7 and FT7, between F5 and FC5, between FC3 and C3, between FC1 and C1 (see FIG. 5(b)), between FCZ and CZ (see FIG. 5(d)), between FC2 and C2, between FC4 and C4, between F6 and FC6, and between F8 and FT8 (see FIG. 5(c)). In some embodiments, the anterosuperior cranial region 500a occupies at least 60%, or at least 70%, or at least 80% of the surface area of the first sub-region. Taking the anterosuperior cranial region 500a as an example, it may be implemented in a block manner enclosing a group of electrode positions (see FIG. 6(a)) or in a connected domain manner with multiple vents (see FIG. 6(b), where vents may correspond to electrode positions or not), so as to achieve a desired percentage relative to the surface area of the first sub-region. In some embodiments, the anterosuperior cranial region 500a may also occupy the entire first sub-region (see FIG. 6(c)) to achieve a irradiation over the first sub-region without dead-angle. This also applies to other regions, such as the cranial vertex region 500b, together with the left lateral cranial region 500c and right lateral cranial region 500d, which will not be repeated herein.

In some embodiments, the cranial vertex region 500b is located in a second sub-region enclosed by a second boundary line 502c. According to the 10-10 standard system, the second boundary line 502c sequentially passes between the following electrode positions: between FC3 and C3, between FC1 and C1, between FCZ and CZ, between FC2 and C2, between FC4 and C4 (see FIG. 5(d)), between C6 and C4, between CP6 and CP4, between P6 and P4 (see FIG. 5(c)), between PO4 and P4, between PO4 and P2, between POZ and P2, between POZ and PZ, between POZ and P1, between PO3 and P1, between PO3 and P3 (see FIG. 5(e)), between P5 and P3, between CP5 and CP3, and between C5 and C3 (see FIG. 5(b)). The cranial vertex region 500b occupies at least 60%, or at least 70%, or at least 80% of the surface area of the second sub-region.

In some embodiments, the left lateral cranial region 500c is located in a third sub-region enclosed by a third boundary line 502d and the total boundary line 501, and the right lateral cranial region 500d is located in a fourth sub-region enclosed by a fourth boundary line 502e and the total boundary line 501. According to the 10-10 standard system, the third boundary line 502d sequentially passes between the following electrode positions (see FIG. 5(b)): between FT7 and F7, between FC5 and F5, between FC5 and FC3, between C5 and C3, between CP5 and CP3, between P5 and P3, between P5 and PO5, and between P7 and PO7. The fourth boundary line 502e sequentially passes between the following electrode positions (see FIG. 5(c)): between FT8 and F8, between FC6 and F6, between FC6 and FC4, between C6 and C4, between CP6 and CP4, between P6 and P4, between P6 and PO6, and between P8 and PO8. Specifically, the left lateral cranial region 500c occupies at least 60%, or at least 70%, or at least 80% of the surface area of the third sub-region, and the right lateral cranial region 500d occupies at least 60%, or at least 70%, or at least 80% of the surface area of the fourth sub-region.

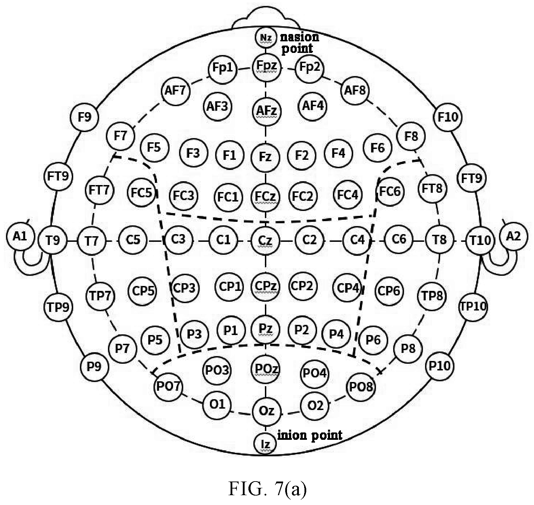

In some embodiments, the anterosuperior cranial region 500a, the cranial vertex region 500b, together with the left lateral cranial region 500c and the right lateral cranial region 500d may be divided according to the sets of electrode positions they include. For example, according to the 10-10 standard system and with reference to FIG. 7(a), each region may include the following electrode positions respectively. The anterosuperior cranial region 500a comprises electrode positions FP2, FPZ, FP1, AF3, AF4, AF7, AF8, AFZ, FZ, F1, F2, F3, F4, F5, F6, F7, F8, FC1, FC2, FC3, FC4, FCZ. The cranial vertex region 500b comprises electrode positions CZ, C1, C2, C3, C4, CPZ, CP1, CP2, CP3, CP4, PZ, P1, P2, P3, P4. The left lateral cranial region 500c comprises electrode positions FT7, FC5, T7, C5, TP7, CP5, P7, P5, and the right lateral cranial region 500d comprises electrode positions FT8, FC6, T8, C6, TP8, CP6, P8, P6.

In some embodiments, each region may include sparser electrode positions, i.e., a contracted coverage range. For example, according to the 10-10 standard system, the anterosuperior cranial region 500a may comprise electrode positions AFZ, FZ, F1, F2, FP1, FP2. The cranial vertex region 500b comprises electrode positions CZ, C1, C2 and CPZ. The left lateral cranial region 500c comprises electrode positions FT7, FC5, T7, C5, or TP7, CP5, P7 or P5, and the right lateral cranial region 500d comprises electrode positions FT8, FC6, T8, C6, or TP8, CP6, P8, P6. Although the coverage range of each region is contracted, by supplying sufficient time-averaged radiant power to each region, the near-infrared light can still break through spatial intervals and propagate to the adjacent core network nodes and brain regions of the default mode network (DMN), central executive network (ECN, also called executive control network), salience network (SN), sensorimotion network (SMN), and dorsal attention network (DAN), thereby performing AD inhibition processing along the propagation paths of functionally connected regions, such as inhibiting or eliminating the deposition of Aβ plaques, inhibiting or eliminating abnormal aggregation of Tau protein, inhibiting or improving neurofibrillary tangles, and thus effectively inhibiting or interrupting the progression of neurodegenerative lesions caused by abnormal Aβ plaques and Tau protein spreading along brain functional networks.

In some embodiments, the anterosuperior cranial region 500a, the cranial vertex region 500b, together with the left lateral cranial region 500c and the right lateral cranial region 500d may be divided in various ways as needed based on the sets of electrode positions they include. Note that such division may be defined for the convenience of irradiation control of the phototherapy device, or for the specific structure of the phototherapy device-in particular, the spatial arrangement of each unit in the array of near-infrared irradiation units. Accordingly, the sets of electrode positions respectively included in the anterosuperior cranial region 500a, the cranial vertex region 500b, together with the left lateral cranial region 500c and the right lateral cranial region 500d may be flexibly divided, as long as they can cover or be sufficiently adjacent to the core network nodes and brain regions of the default mode network (DMN), central executive network (ECN, also called executive control network), salience network (SN), sensorimotion network (SMN), and dorsal attention network (DAN).

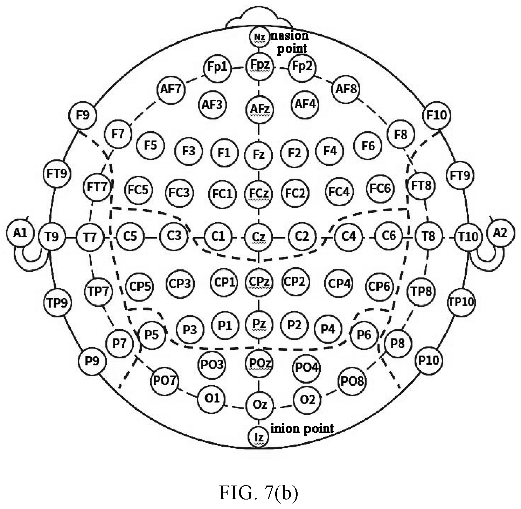

For example, according to the 10-10 standard system, the division of each region may be seen in FIG. 7(b). The anterosuperior cranial region 500a comprises FP2, FPZ, FP1, AF3, AF4, AF7, AF8, AFZ, FZ, F1, F2, F3, F4, F5, F6, F7, FC5, F8, FC1, FC2, FC3, FC4, FCZ, FC6, CZ, C1, C2; the cranial vertex region 500b comprises electrode positions C5, C3, C4, C6, CPZ, CP1, CP2, CP3, CP4, CP5, CP6, PZ, P1, P2, P3, P4. The left lateral cranial region 500c comprises electrode positions FT7, T7, TP7, P7, and the right lateral cranial region 500d comprises electrode positions FT8, T8, TP8, P8.

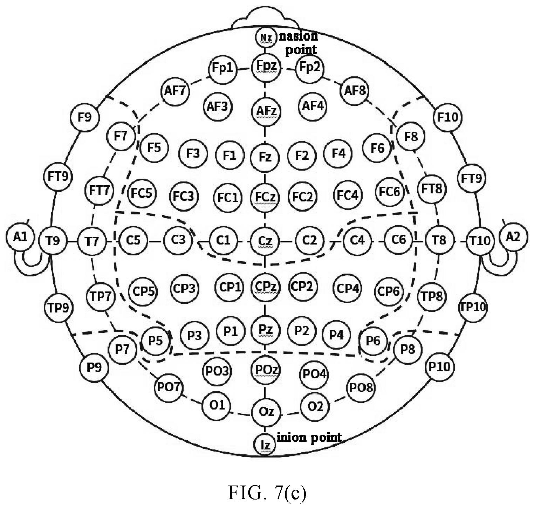

As another example, according to the 10-10 standard system, the division of each region may be seen in FIG. 7(c). The anterosuperior cranial region 500a comprises electrode positions FP2, FPZ, FP1, AF3, AF4, AF7, AF8, AFZ, FZ, F1, F2, F3, F4, F5, F6, FC1, FC2, FC3, FC4, FC5, FC6, FCZ, CZ, C1, C2. The cranial vertex region 500b comprises electrode positions C3, C4, C5, C6, CPZ, CP1, CP2, CP3, CP4, CP5, CP6, PZ, P1, P2, P3, P4. The left lateral cranial region 500c comprises electrode positions F7, FT7, T7, TP7, P5, and the right lateral cranial region 500d comprises electrode positions F8, FT8, T8, TP8, P6.

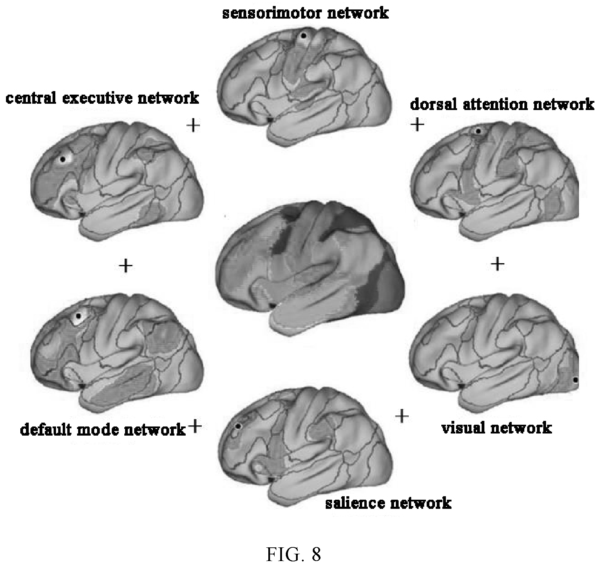

FIG. 8 shows a schematic diagram of the spatial locations of the default mode network (DMN), central executive network (ECN, also called executive control network), salience network (SN), sensorimotion network (SMN), and dorsal attention network (DAN), and Visual Network (VN) in the brain. As shown in FIG. 8, anterior sites of DMN include medial prefrontal cortex, anterior cingulate, dorsolateral prefrontal cortex, and lateral temporal lobe, while posterior sites include medial temporal lobe, hippocampus, angular gyrus, inferior parietal lobule, posterior cingulate, and ventral prefrontal cortex. More than a decade before identifiable cognitive states appear, amyloid-β (Aβ) has begun to deposit significantly in DMN and spreads along functionally connected regions of DMN rather than along spatially correlated but poorly connected sites. Brain functional networks of patients in the dementia stage and MCI stage are often impaired at those nodes such as posterior cingulate, medial temporal lobe (especially hippocampal node), and posterior parietal lobe. Aβ in the brain of MCI patients first deposits in the posterior cingulate, prefrontal lobe, precuneus, and parietotemporal regions, reducing and weakening functional connectivity between these regions and other brain areas. The sites where these lesions occur are fully covered by the anterosuperior cranial region 500a, the cranial vertex region 500b, the left lateral cranial region 500c, and the right lateral cranial region 500d.

The salience network (SN), also called ventral attention network, and the dorsal attention network (DAN) together constitute the attention network (AN). The Salience Network is mainly distributed in the anterior cingulate, frontoinsular cortex, ventral prefrontal cortex, etc. Abnormally increase of the internal functions are associated with impaired functional networks, causing abnormally active psychiatric symptoms in AD patients, especially in the dementia stage, such as but not limited to irritability, abnormal motion behavior (constant walking), easy anger, and aggressive behavior. In fact, weakened specific functional connectivity of DMN leads to compensatory increases in SN-related functional connectivity, such as enhanced SN connectivity of the right pregenual anterior cingulate. DAN is mainly distributed in the intraparietal sulcus and frontal eye regions. Reduced functional connectivity between DAN and SN is also closely related to attention deficits in AD patients. Specifically, both DAN and SN are impaired in AD patients, while in MCI patients, DAN function is impaired but internal functional connectivity of SN is partially preserved. Referring to FIG. 8, the anterosuperior cranial region 500a, the cranial vertex region 500b, the left lateral cranial region 500c, and the right lateral cranial region 500d sufficiently cover the frontal lobe, especially adjacent to the temporal lobe, as well as the parietal lobe, thereby sufficiently covering important lesioned sites in SN and DAN of AD patients.

The central executive network (ECN, also called executive control network) is mainly distributed in the dorsolateral prefrontal cortex, ventral prefrontal cortex, medial prefrontal cortex, parietal cortex, etc., as shown in FIG. 8. In the early stage of AD course, the functional connectivity pattern of ECN has already changed, such as reduced functional connectivity between the left frontal cortex and other brain regions and decreased functional connectivity of the left parietal cortex, which are associated with visuospatial disorders. For example, patients in the dementia stage and MCI stage show significantly reduced functional connectivity in the right frontal lobe and superior frontal gyrus in ECN, which is associated with cognitive decline. Besides, not only internal functional connectivity in ECN decreases, but also functional connectivity between ECN and DMN decreases, leading to the spread of impairments in executive control functions. The anterosuperior cranial region 500a, the cranial vertex region 500b, the left lateral cranial region 500c, and the right lateral cranial region 500d can sufficiently cover these lesioned sites.

The Sensorimotion Network (SMN) governs body sensation and motion, mainly including the precentral gyrus and postcentral gyrus. Referring to FIG. 8, it is mainly distributed in adjacent regions anterior and posterior to the central sulcus and extends bilaterally along the central sulcus toward the temporal lobe. Impaired functional connectivity of SMN causes motion disorders in AD patients. In addition, for patients in the dementia stage and MCI stage, reduced functional connectivity between SMN and DAN is also closely related to attention deficits; as the course of AD progresses, internal functional connectivity of SMN worsens progressively, and functional connectivity with DAN also worsens progressively. The anterosuperior cranial region 500a and the cranial vertex region 500b tightly cover adjacent regions anterior and posterior to the central sulcus, and together with the left lateral cranial region 500c and the right lateral cranial region 500d, extend longitudinally directly to the temporal lobe, sufficiently covering these lesioned sites.

Unlike phototherapy devices lacking cranial vertex coverage or those focusing only on DMN, the phototherapy device of the present application ensures that sufficient time-averaged radiant power is comprehensively applied (delivered) to core lesioned sites of the default mode network (DMN), central executive network (ECN, also called executive control network), salience network (SN), sensorimotion network (SMN), and dorsal attention network (DAN). Furthermore, the phototherapy device of the present application finely divides the anterosuperior cranial region 500a, the cranial vertex region 500b, the left lateral cranial region 500c, and the right lateral cranial region 500d according to the 10-10 standard system, ensuring precise positioning to sites at the junctions of brain regions in the above networks and interconnected sites between different functional networks, thereby reducing missing or omitted sites in the networks. Accordingly, for the near-infrared irradiation by the phototherapy device of the present application, a smoother propagation path along the functionally connected regions is provided, and the propagation and transmission effects of inhibitory responses are more efficient, achieving optimized AD inhibition effects across the whole brain. If the process of delivering irradiation of near-infrared light to the subject's head is analogized to battery charging, e.g., referred to as a "optical-charging" process, the phototherapy device according to the present application, by means of sufficient "optical-charging" with respect to the core sites of DMN, ECN, SN, SMN, and DAN where AD lesions may occur, enables a greater "optical-charging capacity", deeper "optical-charging depth", faster "optical-charging speed" in the subject's brain, and better "runtime per charge". That is, biochemical reactions continue to be triggered after phototherapy interruption, achieving sustained and cumulative benefits. This effect has also been validated by clinical trial results, which will be described in details below.

In some embodiments, the irradiation spatial range further covers the posterior cranial region 500e, wherein the posterior cranial region 500e is located in a fifth sub-region divided from the total region by the fifth boundary line 502a, and the fifth sub-region is located below the first region. Referring to FIG. 5(e), the region above the fifth boundary line 502a is the first region, and the region below the fifth boundary line 502a is the fifth sub-region. The posterior cranial region 500e may occupy the entire fifth sub-region as shown in FIG. 5(e), or only part of the fifth sub-region.

In some embodiments, the posterior cranial region 500e may also be defined by electrode positions it includes; for example, the posterior cranial region 500e may include electrode positions PO7, PO5, PO3, POZ, PO4, PO6, PO8, O1, OZ, and O2.

In some embodiments, the posterior cranial region 500e may also be defined by anatomical feature points; for example, the posterior cranial region 500e may at least comprise the occipital region above the inion.

In some embodiments, the time-averaged total radiant power directly incident upon the calvarial region of the subject's head may be higher as needed, such as but not limited to at least 31 W, or at least 40 W, or at least 60 W, or at least 70 W, or even higher, but the spatiotemporal-average irradiance over the surface of the calvarial region shall not exceed 230 mW/cm² to avoid thermal damage to brain tissue.

The phototherapy device of FIGS. 1-3 is an example of a loose-designed headgear. Specifically, its support mechanism comprises a hood disposed in the air, wherein the hood is shaped to form a cavity. When the subject's head is positioned in place in the accommodating space, the cavity is sized to accommodate the subject's head in a loose manner without applying pressure to the subject's head. This provides greater freedom and comfort during phototherapy for subjects who cannot tolerate confined spaces. Each near-infrared irradiation unit in a first group of near-infrared irradiation units has a preset emission angle, and has a preset spacing and height difference configuration with other near-infrared irradiation units in the first group, such that multiple convergence regions are formed above the cranial vertex region and the anterosuperior cranial region, for converging near-infrared light from several near-infrared irradiation units with different incident angles at each convergence region. Comparing FIGS. 1-3 and FIG. 5(d), the supracranial region includes both the cranial vertex region and part of the anterosuperior cranial region, and is not blocked by other parts of the head. In some embodiments, for black or dark hair, a light-guiding comb may be used to part and then bundle hair on the skull to further improve light transmittance.

In some embodiments, the corresponding near-infrared irradiation units above the cranial vertex region and the anterosuperior cranial region are arranged along an arched curved surface. When the subject's head is positioned in place in the accommodating space, the corresponding near-infrared irradiation units 5 are disposed at a first distance above the cranial vertex region and the anterosuperior cranial region, and the average of the first distance is greater than a predetermined average distance, such that the spatiotemporally averaged irradiance over the convergence regions above the cranial vertex region and the anterosuperior cranial region is at a high level, such as at least 58 mW/cm², or at least 60 mW/cm², or at least 62 mW/cm², or at least 64 mW/cm², or at least 66 mW/cm², or at least 68 mW/cm², or at least 70 mW/cm², or at least 72 mW/cm², etc.

The phototherapy device of FIGS. 1-3 is also an application example of array of near-infrared irradiation units arranged circumferentially at multiple heights. Specifically, the support mechanism comprises a hood disposed in the air, wherein the hood is shaped to form a cavity, and the hood supports at least three groups of near-infrared irradiation units at different heights. Each group is disposed at a corresponding height, and the near-infrared irradiation units within a group are circumferentially spaced at a common (corresponding) height. Such circumferential spacing arrangement ensures stable therapeutic effects when the subject's head rotates in place and maintains balanced and stable therapeutic effects on sites and regions at different circumferential positions of the head.

Unlike designs that enclose the subject's head-neck junction in the hood, the headgear shown in FIGS. 1-3 adopts a "cap-like" structure rather than a "helmet-like" structure. Specifically, the cavity formed by the hood has an increasing cross-sectional area downward. When the subject's head is positioned in place in the accommodating space, the hood is configured that its bilateral lower part align with the tragus or its periphery (e.g., within 5 cm² around the tragus) on both sides, its posterior edge is positioned near the inion (e.g., within a predetermined distance from the inion, e.g., predetermined distance of 2 cm), and its anterior edge extends above the brow ridge. Thus, the subject's sight is unobstructed, and sufficient distance is maintained between the inion (with the largest diameter) and the inner wall, resembling wearing a cap rather than being sealed off in a headgear, reducing psychological pressure on the subject. The downward-expanding cavity prevents the subject from hitting the inner wall when the body slides down due to fatigue or the head suddenly lifts up, thereby avoiding local high temperatures caused by blocking air outlets inn the inner wall and contact with high-temperature regions on the inner wall.

Structure of FIGS. 1-3 shows an example in which the hood includes a light-transmissive protective portion. The hood includes a light-transmissive protective portion; for example, the inner housing 3 is made of light-transmissive material to form the light-transmissive protective portion. The light-transmissive protective portion forms an anterior upper part, a top part, a rear part, a left side part, and a right side part, and encloses the cavity. With the light-transmissive protective portion, the subject's head may be protected from direct contact with the near-infrared irradiation units. In some embodiments, the anterior upper part is arranged corresponding to the anterosuperior cranial region, the top part is arranged corresponding to the cranial vertex region, the rear part is arranged corresponding to the posterior cranial region, the left side part is arranged corresponding to the left lateral cranial region, and the right side part is arranged corresponding to the right lateral cranial region. In some embodiments, near-infrared light emergent from the top part, the anterior upper part, the rear part, the left side part, and the right side part of the light-transmissive protective portion forms an irradiation curved surface without non-irradiated regions on the surface of the calvarial region of the subject's head. In this way, there is no irradiation dead angles for the near-infrared beams emitted from the array of near-infrared irradiation units and emergent via the light-transmissive protective portion, delivering thorough irradiation to all parts of the subject's calvarial region. The irradiation of near-infrared light not only applies transcranially and comprehensively to core lesioned sites of the default mode network (DMN), central executive network (ECN, also called executive control network), salience network (SN), sensorimotion network (SMN), and dorsal attention network (DAN), but also ensures sufficient irradiation to be delivered to sites on functionally connected paths between these core sites.

For example, the light-transmissive protective portion may be an integral housing, and near-infrared light is emergent from inner walls at each positions of the light-transmissive protective portion.

Structural examples of the phototherapy device (especially the headgear) are described below in conjunction with FIGS. 1-3. Note that the headgear structure of FIGS. 1-3 is merely shown as an example. The phototherapy device of the present application may adopt different structures. Various structures of the phototherapy device defined in the claims, as well as descriptions of various components and parts of the phototherapy device in the specification, may independently or freely combine with each other to form embodiments of the phototherapy device of the present application, which are not repeated here.

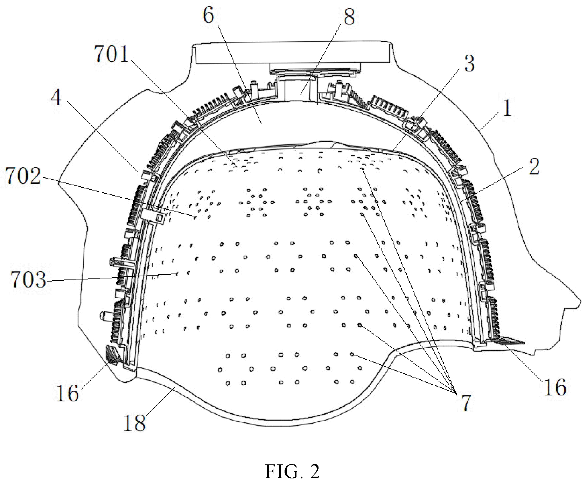

As shown in FIGS. 1 and 2, an embodiment of the present application provides a headgear for a phototherapy device, comprising an outer housing 1, a middle housing 2, and an inner housing 3 arranged sequentially from outside to inside. A irradiation panel accommodating cavity 4, provided with multiple irradiation panels 5 therein, is formed between the outer housing 1 and the middle housing 2, a cold air cavity 6 is formed between the middle housing 2 and the inner housing 3, and the inner housing 3 encloses inward to form an accommodating space. In some embodiments, each irradiation panel may include 4-9 near-infrared LEDs, each with a time-averaged optical power of 80 mW-100 mW, and each near-infrared irradiation unit has an irradiation surface area of 5-9 cm² at its emergent surface.

In some embodiments, the phototherapy device may further include a cooling mechanism configured such that, when the time-averaged total radiant power incident upon the surface of the subject's head reaches 27.5-120 W and the duration of a single continuous irradiation (a session) reaches 30 minutes, the temperature of air in the space adjacent to but not in contact with the subject's head does not exceed 41°C.The cooling mechanism may adopt various structures. The cooling mechanism in the phototherapy device of the present application introduces cold air from the outside and achieves efficient cooling through gas convection and heat transmission driven by the temperature difference between the cold air and the thermal environment around the subject's head in the accommodating space. Taking FIGS. 1-3 as an example, the cooling mechanism may adopt a design that introduces cold air from the outside into the accommodating space via the cold air cavity 6 and ventilation holes 7. It should be understood that other cooling mechanisms are also possible.

Multiple ventilation holes 7 are opened in the inner housing 3 to allow cold air from the cold air cavity 6 to enter the accommodating space via the ventilation holes 7. The ventilation holes 7 may be opened in a first region of the inner housing 3 near the head top and a second region below the first region. In the second region, multiple ventilation hole units are opened layered from top to bottom. The arrangements of upper-layer ventilation hole units and lower-layer ventilation hole units are different, so as to achieve cooling effect with respect to each region of the patient's head, timely cooling of each brain region, improved cooling synchronization, and more balanced temperature perception across the entire head during phototherapy of the patients, thereby enhancing patient comfort when wearing the headgear for phototherapy.

The inner housing 3 is the housing layer closest to the patient's head when the phototherapy device is worn by the patients. The cold air cavity 6 is formed between the middle housing 2 and the inner housing 3, and the irradiation panel accommodating cavity 4 is formed between the outer housing 1 and the middle housing 2. The inner housing 3 and middle housing 2 are configured to be light-transmissive, so that light emitted by the irradiation panels 5 can sequentially pass through the light-transmissive middle housing 2 and inner housing 3 into the accommodating space for phototherapy of the patient.

In this embodiment, the inner housing 3 includes two parts: a first region near the head top and a second region below the first region. The ventilation hole 7 may be opened in both the first and second regions. As shown in FIG. 3, the middle housing 2 is provided with a cold air inlet 8 communicating with the cold air cavity 6. A circle of ventilation holes may be provided at least on the outer edge of the first region to reserve a cavity for a protective pad arranged on the top of the head. In this way, after entering the cold air cavity 6 via the cold air inlet 8, cold air is blown toward the patient's head through the ventilation hole 7, ensuring good cooling experience at the head periphery and head top of the patients during phototherapy. In addition, the above structure enables cold air to flow from the top down along the patient's head, which improves heat exchange efficiency in the accommodating space and makes the temperature in the headgear accommodating space more uniform.

In some embodiments, the cold air inlet 8 of the cold air cavity 6 is provided in the first region and closer to the rear of the inner housing 3 than to the front of the inner housing 3, wherein the front of the inner housing 3 is the direction corresponding to the forehead of the headgear, and the rear of the inner housing 3 is the direction corresponding to the back of the head of the headgear. As shown in FIGS. 1 and 2, the cold air inlet 8 shown in FIG. 2 is closer to the rear of the inner housing 3. The cold air inlet 8 is provided closer to the rear of the inner housing 3 than to the front of the inner housing 3 to make the distances from the cold air inlet 8 to the end of the front of the inner housing 3 (edge position of the front of the inner housing 3) and from the cold air inlet 8 to the end of the rear of the inner housing 3 (edge position of the rear of the inner housing 3) similar, thereby avoiding delayed cooling in some regions and uneven overall cooling caused by excessive differences between distance from the cold air inlet 8 to edge position of the front of the inner housing 3 and distance from the cold air inlet 8 to edge position of the rear of the inner housing 3 due to improper positioning of the cold air inlet 8.

In other embodiments of the present application, to ensure that the cold air inlet 8 can uniformly transmit cold air to the accommodating space through the ventilation holes 7 of the cold air cavity 6, the headgear for a phototherapy device may include multiple cold air inlets 8 distributed at different positions of the inner housing to supply cold air into the cold air cavity 6, thereby improving overall cooling uniformity of the headgear. The position arrangement of the cold air inlet 8 may be adjusted accordingly based on the specific headgear structure and arrangement of phototherapy irradiation panels.

In the present application, both the first region of the inner housing 3 and the second region below it are provided with ventilation holes 7, helping to lower the temperature at the patient's head top and head periphery. In addition, adjusting the position/arrangement number of cold air inlets 8 to dispose them in the first region ensures effective cooling at the head top position. Besides, the arrangement position of cold air inlets 8 being closer to the rear of the inner housing 3 than to the front of the inner housing 3 makes the time for cold air to reach the ends of the front and rear of the inner housing 3 similar, enabling coordinated cooling of each brain region and more balanced temperature perception across the entire head during phototherapy of the patients. The forehead region is not excessively cooled, while the back of the head achieves better cooling effects, further improving patient comfort when wearing the headgear for phototherapy.

As shown in FIGS. 2 and 3, in some embodiments, the second region is provided with multiple ventilation hole units layered from top to bottom. The upper layer of ventilation hole units 701 and lower layer of ventilation hole units 703 have different arrangement manners, while the upper layer of ventilation hole units 701 and middle layer of ventilation hole units 702 have the same arrangement manners. The number of ventilation holes in a single lower layer of ventilation hole unit 703 is smaller than that in a single upper layer of ventilation hole unit 701 or a single middle layer of ventilation hole unit 702, and ventilation holes 7, with a ventilation hole arrangement density relatively lower than that within the upper layer of ventilation hole units 701, are provided between adjacent upper layers of ventilation hole units 701. This arrangement allows the upper layer of ventilation hole units 701 and middle layer of ventilation hole units 702 to release more cold air relatively than the lower layer of ventilation hole units 703.

In some embodiments, the upper layer of ventilation hole units 701 and middle layer of ventilation hole units 702 may each consist of uniformly distributed inner-ring ventilation holes 7 and uniformly distributed outer-ring ventilation holes 7, e.g., six uniformly distributed inner-ring ventilation holes 7 and six uniformly distributed outer-ring ventilation holes 7, while the lower layer of ventilation hole units 703 may consist of only a ring of uniformly distributed ventilation holes 7, e.g., merely six uniformly distributed ventilation holes 7.

In some embodiments, ventilation holes 7 with a ventilation hole arrangement density relatively lower than that within the upper layer of ventilation hole units 701 are further provided between adjacent upper layers of ventilation hole units 701. As shown in FIG. 3, he ventilation holes 7 between the upper layers of ventilation hole units 701 are relatively sparse. No additional ventilation holes 7 are provided between the middle layers of ventilation hole units 702 or between the lower layer of ventilation hole units 703.

When a patient wears the headgear for phototherapy, the air permeability of the headgear varies from top to bottom. The skin corresponding to the patient's head top is in the accommodating space which is a relatively closed space, while the skin corresponding to the patient's back of the head is close to the communication position between the accommodating space and the outside, facilitating air exchange. Therefore, more ventilation holes are provided at positions with poor air fluidity to increase cooling speed, while relatively fewer ventilation holes are provided at positions with well air fluidity to facilitate heat dissipation using the external environment. Based on the principle that cold air moves downward and hot air moves upward, optimal cooling effect of the patient's head is achieved under the premise of releasing equal amounts of cold air through the cold air cavity into the accommodating space. The temperature from top to bottom can be balanced as much as possible through the arrangement of the ventilation hole units, and the comfort during phototherapy can be increased. Furthermore, the distance between adjacent ventilation hole units in each layer of ventilation hole units may be the same.

In some embodiments, ventilation holes 7 are provided at least at positions corresponding to the irradiation panels 5. During phototherapy of the patient, the irradiation panels 5 correspond to the locations where the patient's skin is irradiated, and heat tends to accumulate due to high light intensity. Disposing ventilation holes 7 on the inner housing 3 at least at positions corresponding to the irradiation panels 5 may prevent excessive heat accumulation caused by the irradiation panels 5 irradiating the patient's skin and efficiently transfer the accumulated heat.

In some embodiments, the density of ventilation holes 7 at positions corresponding to the irradiation panels 5 is higher than that at positions without irradiation panels 5. This helps balance the temperature between positions with and without irradiation panels 5, effectively enhances air permeability of the accommodating space of the headgear, improves air heat exchange efficiency in the accommodating space, and further improves patient wearing comfort.

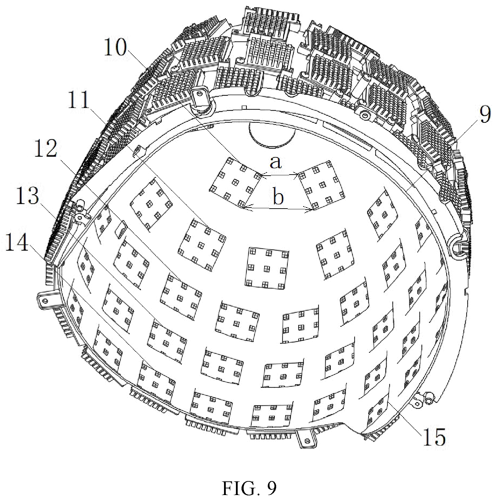

In some embodiments, multiple irradiation panels 5 are provided on the middle housing 2 through a irradiation panel fixing housing 9, which is provided to cover the side of the middle housing 2 close to the outer housing 1. As shown in FIG. 9, in some embodiments, the irradiation panel fixing housing 9 is provided in the irradiation panel accommodating cavity 4 and the irradiation panel fixing housing 9 is provided on the side of the middle housing 2 close to the outer housing 1. Multiple irradiation panels 5 are arranged layer by layer from top to bottom along the irradiation panel fixing housing 9, and the distance between two adjacent irradiation panels 5 on the upper layer is larger than that between two adjacent irradiation panels 5 on the lower layer, ensuring delivery of effective time-averaged radiation energy to all parts of the surface of the calvarial region of the subject's head, i.e., the anterosuperior cranial region, the cranial vertex region, the left lateral cranial region, together with the right lateral cranial region (and optionally the posterior cranial region), thereby delivering sufficient and effective time-averaged radiation energy transcranially to core sites and regions of the subject's brain functional networks.

Specifically, the spatiotemporally averaged irradiance over (i.e., irradiated to) the cranial vertex region and the anterosuperior cranial region is at least 55 mW/cm², and the spatiotemporally averaged irradiance irradiated to the left lateral cranial region and the right lateral cranial region is at least 35 mW/cm². In some embodiments, it can be understood that when multiple irradiation panels 5 are distributed on a spatial curved surface, which covers the subject's head with a predetermined gap, the spatiotemporally averaged irradiance obtained based on the time-averaged irradiance at each representative position of the cranial vertex region and the anterosuperior cranial region is higher than that obtained based on the time-averaged irradiance at each representative position of the left lateral cranial region and the right lateral cranial region. This is particularly applicable to the circumferential multi-height array of near-infrared irradiation units and loose headgear design shown in FIGS. 1-3. Specifically, although such a design has high acceptance among AD patients who dislike restraint, the left lateral cranial region and the right lateral cranial region are easily blocked or show weak irradiation superposition effects, leading to low irradiance. By enabling the cranial vertex region (where near-infrared light can sufficiently converge) and the anterosuperior cranial region (with less hair and no blocking) to bear as much irradiance as possible, benefiting from the largest surface areas of these two regions among the several regions, a larger overall power can be delivered to the anterosuperior cranial region, the cranial vertex region, the left lateral cranial region, together with the right lateral cranial region. Moreover, the anterosuperior cranial region and the cranial vertex region correspond to many core sites and regions of brain functional networks. In this way, it is possible to achieve efficient "optical-charging" of core sites and regions of brain functional networks prone to AD lesions. The applied irradiated energy of near-infrared light ensures a smoother propagation path along the functionally connected regions, and the propagation and transmission effects of inhibitory responses are more efficient, achieving optimized AD inhibition effects across the whole brain.

In some embodiments, the posterior cranial region may also be optionally included in the irradiation spatial range, and the requirement for the spatiotemporally averaged irradiance over the posterior cranial region may be lowered, e.g., 1-30 mW/cm² is sufficient. Through extensive irradiation tests, the inventors found that the time-averaged irradiance at different positions on the posterior cranial region offset more obviously. Referring to the structure of FIGS. 1-3, especially for a loose headgear design, the posterior cranial region corresponds to the occiput, which is located at a low position and has a concave curved shape. Such a three-dimensional structure easily blocks near-infrared light directed thereto, and the three-dimensional height fluctuations of various positions cause variable blocking, e.g., the time-averaged irradiance at various positions may be as low as several mW/cm² or as high as 30 or even 40 mW/cm², resulting in unstable spatiotemporally averaged irradiance over the posterior cranial region. In view of this, in the headgear structure of the phototherapy device of the present application, even if the posterior cranial region is covered, the spatiotemporally averaged irradiance over (i.e., irradiated to) the cranial vertex region and the anterosuperior cranial region, as well as that irradiated to the left lateral cranial region and the right lateral cranial region, are both (significantly) higher than that irradiated to the posterior cranial region. That is, the posterior cranial region with variable blocking is made to bear as little spatiotemporally averaged irradiance and total radiant power as possible, while still ensuring the AD inhibition or prevention effect for the subject.

In some embodiments, the representative positions comprise at least one of the following: electrode positions according to the 10-10 standard system contained within each region, or a predetermined proportion thereof; electrode positions according to the 10-10 standard system contained within each region and non-electrode positions among the electrode positions, or a predetermined proportion thereof; a predetermined number of positions selected in each region according to a predetermined manner. The predetermined manner for selecting comprises at least one of the following: randomly selecting a predetermined number of positions; uniformly dividing each region into a predetermined number of grids and selecting the predetermined number of positions corresponding to the grids; selecting the predetermined number of positions excluding edge positions; selecting positions of projection points in each region for the centers of the near-infrared irradiation units.

In other embodiments of the present application, multiple irradiation panels 5 may be directly provided on the middle housing 2.

In an embodiment of the present application, as shown in FIG. 9, six layers of irradiation panels 5 are arranged at intervals on the irradiation panel fixing housing 9, and the irradiation panels 5 comprise at least one of the following arrangement modes.

Mode 1: In the first layer of irradiation panels 10 closest to the top of the headgear, two adjacent irradiation panels have a first gap, the length of the narrowest position a of the first gap ranging from 23 mm to 26 mm, and the length of the widest position b of the first gap ranging from 57 mm to 60 mm. Optionally, the length of the narrowest position a of the first gap ranges from 23.5 mm to 25.5 mm, and the length of the widest position b of the first gap ranges from 57.5 mm to 59.5 mm. Preferably, the length of the narrowest position a of the first gap is about 24 mm, and the length of the widest position b of the first gap is about 59 mm.

Mode 2: In the second layer of irradiation panels 11 adjacent to the first layer of irradiation panels 10, two adjacent irradiation panels have a second gap, the length of the narrowest position of the second gap ranging from 15 mm to 18 mm, and the length of the widest position of the second gap ranging from 41 mm to 44 mm. Optionally, the length of the narrowest position of the second gap ranges from 16 mm to 17.5 mm, and the length of the widest position of the second gap ranges from 41 mm to 43 mm. Preferably, the length of the narrowest position of the second gap is about 16.7 mm, and the length of the widest position of the first gap is about 42.5 mm.

Mode 3: In the third layer of irradiation panels 12 below the second layer of irradiation panels 11, two adjacent irradiation panels have a third gap, the length of the narrowest position of the third gap ranging from 13 mm to 16 mm, and the length of the widest position of the third gap ranging from 24 mm to 27 mm. Optionally, the length of the narrowest position of the third gap ranges from 13.5 mm to 15.5 mm, and the length of the widest position of the third gap ranges from 24 mm to 26 mm. Preferably, the length of the narrowest position of the third gap is about 14.2 mm, and the length of the widest position of the third gap is about 25.3 mm.

Mode 4: Among the fourth layer of irradiation panels 13, fifth layer of irradiation panels 14, and sixth layer of irradiation panels 15 located below the third layer of irradiation panels 12 and arranged sequentially from top to bottom, two adjacent irradiation panels of the fourth layer of irradiation panels 13 have a fourth gap, the length of the narrowest position of the fourth gap ranging from 12 mm to 15 mm, and the length of the widest position of the fourth gap ranging from 19 mm to 22 mm. Optionally, the length of the narrowest position of the fourth gap ranges from 13.5 mm to 14.5 mm, and the length of the widest position of the fourth gap ranges from 19.5 mm to 21.5 mm. Preferably, the length of the narrowest position of the fourth gap is about 14 mm, and the length of the widest position of the fourth gap is about 20.5 mm.

Mode 5: Two adjacent irradiation panels of the fifth layer of irradiation panels 14 have a fifth gap, the length of the narrowest position of the fifth gap ranging from 12 mm to 15 mm, and the length of the widest position of the fifth gap ranging from 16 mm to 19 mm. Optionally, the length of the narrowest position of the fifth gap ranges from 13 mm to 14.5 mm, and the length of the widest position of the fifth gap ranges from 16.5 mm to 18.5 mm. Preferably, the length of the narrowest position of the fifth gap is about 13.8 mm, and the length of the widest position of the fifth gap is about 18 mm.

Mode 6: The sixth layer of irradiation panels 15 is divided into left and right parts, each having 3 irradiation panels. Two adjacent irradiation panels have a sixth gap, the length of the narrowest position of the sixth gap ranging from 11 mm to 14 mm, and the length of the widest position of the sixth gap ranging from 15 mm to 18 mm. Optionally, the length of the narrowest position of the sixth gap ranges from 12 mm to 14 mm, and the length of the widest position of the sixth gap ranges from 15 mm to 17 mm. Preferably, the length of the narrowest position of the sixth gap is about 13 mm, and the length of the widest position of the sixth gap is about 16.2 mm.

The above distance provision of the irradiation panels 5 and settings between adjacent layers of the irradiation panels 5 reduce local overheating in the headgear caused by concentrated heat dissipation of the irradiation panels 5 and save energy consumption.

In a further optional embodiment of the present application, as shown in FIG. 9, the gaps between two adjacent irradiation panels 5 in each layer of irradiation panels 5 each have a narrowest position and a widest position, and the width of the gap between two adjacent irradiation panels 5 on each layer from top to bottom is larger than that on the lower layer. On the premise of ensuring the irradiation by the irradiation panels 5 to achieve the phototherapy effect, it reduces the probability of local overheating in the phototherapy headgear caused by concentrated heat dissipation of the irradiation panels 5 due to dense arrangement of the irradiation panels 5. Wherein, due to the structural design of the phototherapy device headgear, the accommodating space tends to have an inner diameter that gradually increases from top to bottom. Therefore, the arrangement distances of the first layer of irradiation panels 10 and second layer of irradiation panels 11 is increased to ensure uniformity of irradiance while avoiding heat dissipated from the irradiation panels 5 accumulating in the headgear and facilitating cooling. To achieve uniformity of irradiance in the entire accommodating space, the optimal average optical power of the first layer of irradiation panels 10 and second layer of irradiation panels 11 may be 75 mW to 125 mW, preferably about 100 mW.

As shown in FIG. 9, in some embodiments, the vertical distance between the first layer of irradiation panels 10 and the second layer of irradiation panels 11 is 19 mm to 25 mm, preferably 19.5 mm to 24.5 mm. The vertical distance between the second layer of irradiation panels 11 and the third layer of irradiation panels 12 is 14 mm to 21 mm, preferably 15 mm to 20 mm.

As shown in FIG. 9, in some embodiments, the vertical distance between other two adjacent layers of irradiation panels is 13 mm to 19 mm, preferably 14 mm to 18 mm. The vertical distance arrangement between two adjacent layers of irradiation panels can satisfy irradiation of the entire brain region and avoid heat dissipated from the irradiation panels 5 accumulating in the accommodating space.

Therefore, the arrangement of the irradiation panels 5 can ensure that the irradiation is delivered to all regions of the patient's head, including the anterosuperior cranial region, the cranial vertex region, the left lateral cranial region, and the right lateral cranial region, without affecting normal progress of phototherapy, and can also improve the comfort during treatment process.

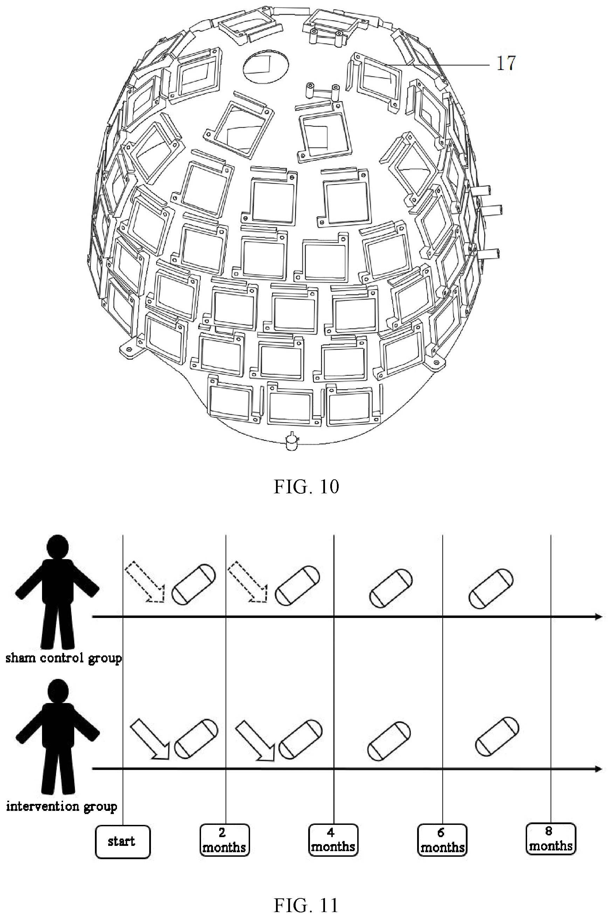

In addition, as shown in FIG. 10, the irradiation panels 5 are connected with irradiation panel fixing frames 17, and the irradiation panels 5 are detachably fixed to the irradiation panel fixing housing 9 via the irradiation panel fixing frames 17. In some embodiments, the distance between adjacent irradiation panel fixing frames 17 in each layer of irradiation panel fixing frames 17 is similar to or the same as the distance between corresponding adjacent irradiation panels 5.

Returning to FIG. 3, in some embodiments, the headgear is provided with an annular connecting part 18. The bottoms of the outer housing 1 and middle housing 2 are respectively connected with the connecting part 18, which is provided with multiple ventilation openings 16 communicating with the irradiation panel accommodating cavity 4. The area of each ventilation opening 16 at the rear end of the headgear is larger than that at the front end of the headgear. Further, the ventilation openings 16 may be elongated shape. This increases the air flow entering the rear end of the headgear, improving the cooling effect of the irradiation panel accommodating cavity 4 and balancing cooling effect at the back of the head and forehead of the headgear. Furthermore, it also reduces heat transmission from the irradiation panel accommodating cavity 4 to the cold air cavity 6, helping to improve the cooling effect on the accommodating space.

In some embodiments, the top of the headgear is provided with an air extraction opening communicating with the irradiation panel accommodating cavity 4 for extracting hot air from the irradiation panel accommodating cavity 4.

In some embodiments, a total of 80 irradiation panels 5 are distributed inside the headgear. The near-infrared light emergent port of each irradiation panel 5 is 28 mm × 28 mm in size, with a tolerance of ±0.2 mm, and the irradiation units are arranged in a 3×3 array.

An embodiment of the present application further provides a phototherapy device comprising a trolley-mounted control device and the headgear according to any embodiment of the present application. The trolley-mounted control device includes a cooling device and an air extraction device. The cooling device is connected with the cold air inlet 8 and configured to supply cold air into the cold air cavity 6 through the cold air inlet 8. The air extraction device is connected with the air extraction opening of the headgear. Phototherapy is performed on the patient through cooperation between the trolley-mounted control device and the headgear, and the comfort of the wearer is improved during phototherapy.

A series of clinical trials were conducted using the phototherapy device of this structure. As an example, the central wavelength of near-infrared light emitted by the array of near-infrared irradiation units is 810 nm, the duty ratio is 50%, and the frequency is 10 Hz, and it should be understood that this is merely an example. In some embodiments, near-infrared light may be emitted with a duty ratio of 30%-70%, a wavelength of 650-1100 nm, and a frequency falling within the Alpha wave frequency range, Gamma wave frequency range, or the neighborhood thereof, which are not repeated here.

In some embodiments, the array of near-infrared irradiation units is specifically configured to deliver irradiation energy of at least 8,260-42,260 Joules to the subject's head within a continuous irradiation time of 5 minutes as a unit energy delivery. Irradiation of 8,260 Joules in 5 minutes is equivalent to continuous irradiation of the calvarial region at a time-averaged radiant power of 27.5 W. Calculating based on a calvarial surface area of 612.35 cm², the spatiotemporally averaged irradiance is 45 mW/cm². Irradiation of 42,260 Joules in 5 minutes is equivalent to continuous irradiation of the calvarial region at a time-averaged radiant power of 140.8 W. Based on a calvarial surface area of 612.35 cm², the spatiotemporally averaged irradiance is 230 mW/cm². The inventors found in clinical trials that it is difficult for some AD patients in moderate and severe dementia stages to sustain irradiation for 10 minutes to half an hour without interruption. Some AD patients with severe cognitive impairment are uncooperative, and some AD patients also frequently exhibit abnormal limb movements, such as muscle stiffness and flexion, muscular atrophy and weakness, apraxia, etc. Using 5 minutes as a unit energy delivery means that each 5-minute irradiation segment achieves an AD inhibition effect to a certain extent.

For example, the required energy delivery (e.g., 3 unit energy deliveries) to the subject's head within a single time period of 1 hour, at least 24,800-126,800 Joules, may be separately administered in discrete or continuous effective unit energy deliveries. The single-session cumulative energy delivery in this way achieves a comparable AD inhibition effect to that delivered for 15 minutes without interruption. During actual treatment process, even if interruption occurs due to the AD patients' own reasons or operational failure, the operator only needs to ensure that the cumulative irradiation time is met within the single-session (i.e., single time period), he/she does not need to restart the phototherapy device, or reset the current irradiation energy delivery to zero, or forcibly interrupting irradiation for fixed rest periods. Specifically, if the subject is cooperative and treatment proceeds smoothly, direct continuous irradiation for 15 minutes may be performed. If the subject is uncooperative or treatment is not smooth, e.g., the subject uses the restroom after 6 minutes of irradiation, treatment may be temporarily interrupted and resumed after the subject returns. The interruption time may be flexibly adjusted according to the subject's needs, as long as all irradiation time is achieved within 1 hour. This significantly reduces the operational difficulty of phototherapy for AD patients with severe cognitive impairment or severe abnormal movements leading to poor cooperation, subject groups with variable severity of AD symptoms (e.g., cross-age nursing home populations), and even caregivers with insufficient experience in caring for AD patients.

In some embodiments, the array of near-infrared irradiation units is specifically configured to deliver irradiation energy of at least 24,800-1,014,100 Joules to the subject's head within a single day, as a daily cumulative energy delivery. It can be seen that the daily cumulative energy delivery may span nearly 40 times, with the lower limit being cumulative irradiation for 15 minutes at a time-averaged radiant power of 27.5 W and the upper limit being irradiation for 2 hours at a time-averaged radiant power of 140.8 W. The inventors found that using the phototherapy device of the present application, the daily cumulative energy delivery may be adjusted over a wide range according to the subject's adaptability. Specifically, if the subject is highly cooperative with phototherapy and shows good individual response, the array of near-infrared irradiation units is specifically configured to implement the single-session cumulative energy delivery by continuous irradiation for more than 30 minutes within a single session of 1 hour, and to implement single-session cumulative energy delivery for the up to 4 times within a single day, to provide an accelerated daily cumulative energy delivery. This accelerated daily cumulative energy delivery is equivalent to the energy delivery implemented for several days with a small daily cumulative energy delivery. If the subject needs to interrupt treatment for several days due to specific circumstances, the accelerated daily cumulative energy delivery may be administered first, in this way, increasing the flexibility and convenience of phototherapy. In addition, although the mechanism of action is unclear, after implementing the accelerated daily cumulative energy delivery to a few individual volunteers, the improvement in their cognitive performance is even better than that achieved by implementing the same cumulative energy delivery in several days.

In some embodiments, the array of near-infrared irradiation units is specifically configured to deliver the irradiation energy of at least 124,000 Joules (27.5 W-15 minutes-5 times) to 7,099,000 Joules (230 mW/cm²-2 hours per day-7 days) to the subject's head within one week using separated single-session cumulative energy deliveries, as a weekly cumulative energy delivery, wherein within each day of the week during which irradiation is performed, the single-session cumulative energy delivery can be implemented in separate time slots for 1, 2, 3, 4, 5, 6, 7, or 8 times.

Specifically, the array of near-infrared irradiation units is specifically configured to implement the energy delivery as much as more than 8 times as the weekly cumulative energy delivery within 8 weeks, preferably implement the energy delivery as much as more than 16 times as weekly cumulative energy delivery within 16 weeks, as a treatment course cumulative energy delivery.

In some embodiments, the treatment course interruption time between two treatment courses does not exceed half of the treatment course duration, so as to avoid deterioration and regression of AD lesions caused by treatment interruption.

The applicant conducted clinical trials for the intervention group (i.e., treatment group) using the phototherapy device shown in FIGS. 1-3. The spatiotemporally averaged irradiance of the phototherapy device over each region of the reference head phantom of the subject's head is shown in Table 1. The phototherapy device in this clinical trial, when the subject's head is positioned in place in the accommodating space formed by the support mechanism, forms an irradiation spatial range covering the anterosuperior cranial region, the cranial vertex region, together with the left lateral cranial region and the right lateral cranial region, as well as posterior cranial region on the surface of the calvarial region of the subject's head. The time-averaged total radiant power incident upon the surface of the calvarial region of the subject's head is at least 31 W, approximately 32 W-48 W, and the spatiotemporally averaged irradiance over each region does not exceed 230 mW/cm².

**Table 1 Spatiotemporally averaged irradiance of the phototherapy device over each region of the reference head phantom**

| Time-averaged irradiance over the upper part of the irradiation surface of inner housing 3 | Time-averaged irradiance over other parts of the irradiation surface of inner housing 3 | Proportion of total area of the anterosuperi or cranial region | Spatiotemporally averaged irradiance over each part of the anterosuperior cranial region | Proportion of total area of the cranial vertex region | Spatiotemporally averaged irradiance over each part of the cranial vertex region |
|---|---|---|---|---|---|
| 50 to 100 mW/cm² | 100 mW/cm², error ≤ ±20% | 35.7% | 50-80 mW/cm² | 23.51% | 60-80 mW/cm² |

| Proportion of total area of the left lateral cranial region | Spatiotemporally averaged irradiance over each part of the left lateral cranial region | Proportion of total area of the right lateral cranial region | Spatiotemporally averaged irradiance over each part of the right lateral cranial region | Proportion of total area of the posterior cranial region | Spatiotemporally averaged irradiance over each part of the posterior cranial region |
|---|---|---|---|---|---|
| 14.17% | 32-60 mW/cm² | 14.17% | 32-60 mW/cm² | 12.45% | 1-30 mW/cm² |

The applicant conducted clinical trials for the intervention group (i.e., treatment group) using the phototherapy device with the operating parameters in Table 2.

**Table 2 Operating parameters of the phototherapy device**

| Central wavelength | Delivered total radiant power | Frequency | Single/dual/triple wavelength | Time-averaged optical power of LED | Daily treatment plan | Days of treatment per week |
|---|---|---|---|---|---|---|
| 810 nm | 40 W ±20% | 10 Hz | Single wavelength | 90-100 mW | Once, 30 minutes | 6 days |

The inclusion criteria for this clinical trial are as follows: (1) meeting the core criteria for probable Alzheimer's disease (AD) as defined by the National Institute on Aging-Alzheimer's Association (NIA-AA); (2) cranium MRI inspection result (within 6 months) supporting a probable AD diagnosis; (3) aged 50-85 years, male or female; (4) MMSE score <26, able to cooperate with scale assessment; (5) patients not taking medication, or if taking psychotropic or cognitive-improving drugs, with stable dosage for at least 12 weeks before the trial and unchanged during treatment.

Exclusion criteria: (1) having MRI contraindications, such as metal implants or claustrophobia; (2) other types of dementia or other psychiatric or neurological diseases, such as depression or Parkinson's disease; (3) history of stroke or epilepsy; (4) photosensitivity to sunlight or visible light, or increased skin sensitivity in the treatment area; (5) severe visual or hearing impairment; (6) history of alcohol or drug addiction; (7) any other conditions unsuitable for participation in this study.

A total of 27 patients were enrolled according to the inclusion and exclusion criterias, including 13 people in the intervention group and 14 people in the sham control group. The intervention group received whole-head near-infrared light stimulation: wavelength of 810 nm, frequency of 10 Hz. Each participant received 30-minute treatment once per day, 6 days per week, for 4 months. The sham control group followed the same protocol as the near-infrared light intervention group but used a sham treatment headgear. The light emitted by the sham treatment device was visually identical to that of the near-infrared treatment device, and produced similar sounds and scalp warmth during treatment with that of the near-infrared treatment device, but with very low optical power that was mostly absorbed by tissue and failed to achieve the effect of stimulating brain tissue (see FIG. 11).

Scale assessments were conducted at the 2nd and 4th months during treatment, and at 2 and 4 months after treatment (6^{th} and 8^{th} months), as shown in FIG. 11. It can be seen that this clinical trial of the present application set the interval between scale statistics to 2 months instead of 1 month, which weakens the learning effect of subjects and makes scale scores more objective and accurate.

Assessors, subjects, and their caregivers were blinded to treatment allocation throughout the study until the end of the study. Furthermore, no discussion of treatment allocation occurred among relevant personnel during the entire study. All subjects believed they received real near-infrared treatment.

Finally, 18 patients (9 patients in the intervention group and 9 patients in the sham control group) completed 4 months of treatment and 4 months of assessment, among whom 1 patient completed only the MMSE scale assessment and did not complete the ADAS-cog scale assessment. The ADAS-Cog scale consists of 12 items covering memory, orientation, language, praxis, attention, etc., and may assess the severity of cognitive symptoms and treatment changes in AD cognitive symptoms, commonly used for therapeutic efficacy evaluation in mild-to-moderate AD (an improvement of 4 points is usually used as the clinical criterion for significant drug efficacy). The MMSE scale is the most widely used cognitive screening scale at home and abroad, covering orientation, memory, attention, calculation, language ability, and visuospatial ability. Studies on MMSE have shown that in specialized clinics such as memory clinics or community hospitals, MMSE achieves a sensitivity and specificity of over 80% in distinguishing normal elderly from dementia, with good value for dementia screening.

To explore the persistence of the therapeutic efficacy of near-infrared light in AD patients, follow-up visits were conducted on the subjects after treatment. A total of 14 subjects completed the 8^{th}-month follow-up (i.e., 4 months after treatment, including 8 subjects in the intervention group and 6 subjects in the sham control group).

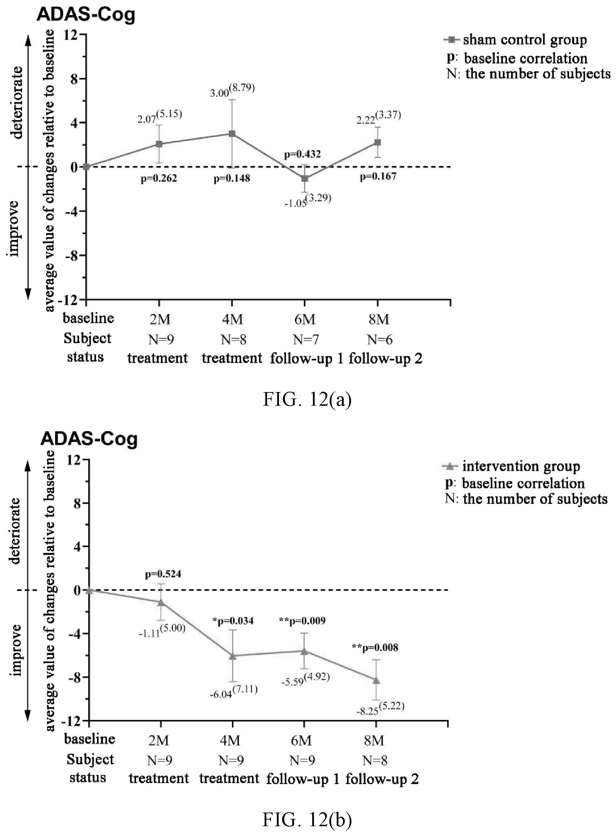

Referring to FIGS. 12(a) and 12(b), it can be seen that after 2 months of phototherapy, the ADAS-cog scale score of subjects in the intervention group decreased by an average of 1.11 points from baseline. After 4 months of treatment, the score continued to decrease at a steeper slope, with an average decrease of 6.04 points from baseline, and the intra-group statistical significance p=0.034<0.05, both significantly better than the scores of the sham control group. Within 2 months after treatment, the ADAS-cog scale score of the intervention group fluctuated slightly, remaining at an average decrease of 5.59 points from baseline at 2 months after treatment, with intra-group statistical significance p=0.009<0.05. At the 2-month follow-up after treatment, the ADAS-cog scale score of the intervention group even began to decrease further, with an average decrease of 8.25 points from baseline at 4 months after treatment, and intra-group statistical significance p=0.008<0.05. From the ADAS-cog scale score, the phototherapy device of the present application achieved beneficial effects never reported in papers and related literatures of other existing phototherapy devices: within 4 months of phototherapy, a steeper decrease in ADAS-cog scale score occurred after the 2^{rd}-month compared with the first 2 months; two follow-ups at 2-month intervals after phototherapy ended showed that the biochemical reactions triggered by near-infrared irradiation energy delivered to the subject's head continued to induce inhibitory effects after phototherapy ended, not only maintaining the AD inhibition effect to a certain extent but even continuously promoting it, without deterioration or regression of the ADAS-cog scale score.

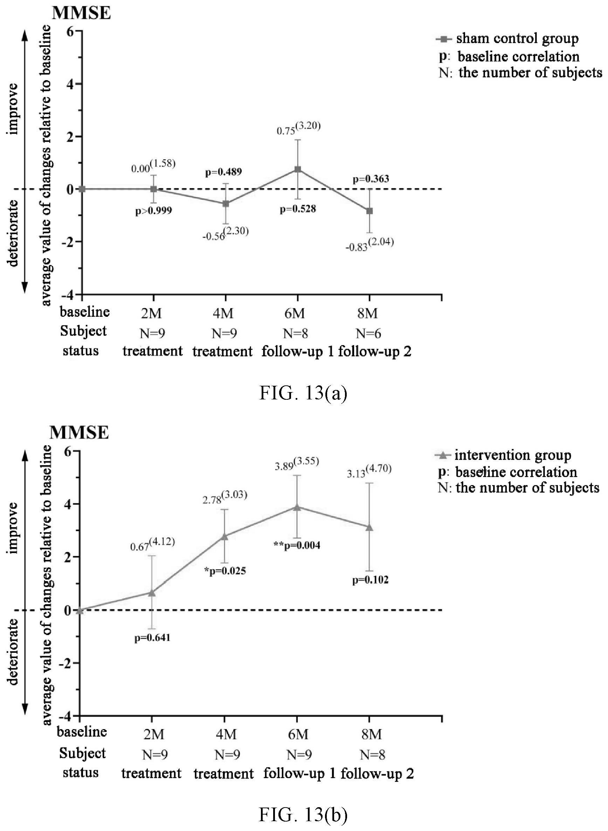

Referring to FIGS. 13(a) and 13(b), it can be seen that after 2 months of phototherapy, the MMSE scale score of subjects in the intervention group increased by an average of 0.67 points from baseline. During the subsequent 2 months of phototherapy, the MMSE scale score continued to increase at a steeper slope, with an average increase of 2.78 points from baseline at 4 months of phototherapy, both significantly better than the scores of the sham control group, and intra-group statistical significance P=0.025<0.05. The ADAS-cog and MMSE scale scores showed similar improvement trends in the first 2 months and the second 2 months of phototherapy: continuous improvement at a steeper slope in the second 2 months. Furthermore, similar to the continuous improvement of ADAS-cog scale score after phototherapy, the MMSE scale score continued to rise for 2 months after phototherapy ended despite the discontinuation of phototherapy, even maintaining a slope comparable to that during phototherapy, with an average increase of 3.89 points from baseline at 2 months after treatment, and intra-group statistical significance p=0.004<0.05. At 4 months after phototherapy, the MMSE scale score of the intervention group remained comparable to that at 4 months of phototherapy, without deterioration or regression. In other words, from the MMSE scale score, the biochemical reactions triggered by near-infrared irradiation energy delivered to the subject's head continued to induce inhibitory effects after phototherapy ended, not only maintaining the AD inhibition effect to a certain extent but even continuously promoting it, without deterioration or regression of the ADAS-cog scale score.

Combined with the results of ADAS-Cog and MMSE scale scores during and after phototherapy follow-up, the optimized "optical-charging" and sustained benefit process of the phototherapy device of the present application mentioned above is also validated: a larger "optical-charging capacity", deeper "optical-charging depth", and faster "optical-charging speed", for the subject's brain. The scale scores improved significantly in both the first 2 months and the second 2 months of phototherapy, progressing at a stable slope without stagnation. The subsequent "runtime per charge" and sustained benefits are better, with the effect of 2 months of phototherapy maintained for at least 2 months after phototherapy ends, even up to at least 4 months after phototherapy ends. The biochemical reactions caused by "optical-charging" continue to trigger inhibitory effects after phototherapy ends and continuously promote AD inhibition without deterioration or regression.

Furthermore, after 4 months of near-infrared phototherapy, resting-state functional magnetic resonance imaging of subjects in the intervention group showed enhanced ALFF in multiple brain regions of the frontal, occipital, and temporal lobes (P<0.05), indicating increased neuronal excitability and spontaneous activity in subjects, providing neuroimaging evidence for corresponding improvements in cognitive function. Meanwhile, no adverse events related to the test device were observed in this trial.

Although phototherapy lasted for 4 months in this clinical trial, it showed characteristics of deep modulation, sustained benefits, and even cumulative benefits. It is expected that benefits will continue to increase with longer continuous use, such as 6 months, 8 months, 10 months ... more than 1 year, or even perennial use, showing a more significant disease-modifying effect.

The above description is intended to be illustrative rather than restrictive, and those of common skill in the art may make changes, modifications, substitutions, and variations to the above embodiments within the scope of the present disclosure. Moreover, the above examples (or one or more solutions thereof) may be used in combination with each other, and it is contemplated that these embodiments may be combined with each other in various combinations or permutations.

## Claims

1. A phototherapy device for inhibiting or preventing Alzheimer's disease, **characterized by** comprising:
a support mechanism, which is configured to form an accommodating space for a subject's head and to support an array of near-infrared irradiation units; and
the array of near-infrared irradiation units, which are configured to emit near-infrared light into the accommodating space, forming, when the subject's head is positioned in place in the accommodating space, an irradiation spatial range that covers at least the anterosuperior cranial region, the cranial vertex region, together with the left lateral cranial region and the right lateral cranial region on a surface of a calvarial region of the subject's head; wherein the time-averaged total radiant power incident upon the surface of the calvarial region of the subject's head is at least 27.5 W, or at least 31 W, or at least 40 W, or at least 60 W, or at least 70 W, and the spatiotemporally averaged irradiance over each irradiated region does not exceed 230 mW/cm².

2. The phototherapy device according to claim 1, **characterized in that** the subject's head comprises the head of a treatment subject, or a reference head phantom of a population of treatment subjects, wherein the head of the treatment subject or the reference head phantom of the population of treatment subjects has the following parameters: a head width of 140-166 mm, a head length of 170-196 mm, and a head circumference of 525-583 mm.

3. The phototherapy device according to claim 1, **characterized in that** the anterosuperior cranial region, the cranial vertex region, the left lateral cranial region, the right lateral cranial region, together with the posterior cranial region are located within a total region enclosed by a total boundary line, the total boundary line passes from a glabella point of the subject's head through the preauricular points on both sides, and extends posteriorly around the head, passing between the inion and the electrode positions O1, OZ, and O2 of the 10-10 standard system to converge; the anterosuperior cranial region, the cranial vertex region, together with the left lateral cranial region and the right lateral cranial region are located within a first region divided from the total region by a fifth boundary line, and occupy at least 60%, or at least 70%, or at least 80% of the surface area of the first region;
according to the 10-10 standard system, the fifth boundary line sequentially passes between the following electrode positions: between P7 and PO7, between P5 and PO5, between P3 and PO3, between P1 and POZ, between PZ and POZ, between P2 and POZ, between P4 and PO4, between P6 and PO6, and between P8 and PO8.

4. The phototherapy device according to claim 3, **characterized in that**,
the anterosuperior cranial region is located in a first sub-region enclosed by a first boundary line and the total boundary line; according to the 10-10 standard system, the first boundary line sequentially passes between the following electrode positions: between F7 and FT7, between F5 and FC5, between FC3 and C3, between FC1 and C1, between FCZ and CZ, between FC2 and C2, between FC4 and C4, between F6 and FC6, and between F8 and FT8; and the anterosuperior cranial region occupies at least 60%, or at least 70%, or at least 80% of the surface area of the first sub-region; and/or
the cranial vertex region is located in a second sub-region enclosed by a second boundary line; according to the 10-10 standard system, the second boundary line sequentially passes between the following electrode positions: between FC3 and C3, between FC1 and C1, between FCZ and CZ, between FC2 and C2, between FC4 and C4, between C6 and C4, between CP6 and CP4, between P6 and P4, between PO4 and P4, between PO4 and P2, between POZ and P2, between POZ and PZ, between POZ and P1, between PO3 and P1, between PO3 and P3, between P5 and P3, between CP5 and CP3, and between C5 and C3; and the cranial vertex region occupies at least 60%, or at least 70%, or at least 80% of the surface area of the second sub-region; and/or
the left lateral cranial region is located in a third sub-region enclosed by a third boundary line and the total boundary line, and the right lateral cranial region is located in a fourth sub-region enclosed by a fourth boundary line and the total boundary line; according to the 10-10 standard system, the third boundary line sequentially passes between the following electrode positions: between FT7 and F7, between FC5 and F5, between FC5 and FC3, between C5 and C3, between CP5 and CP3, between P5 and P3, between P5 and PO5, and between P7 and PO7; the fourth boundary line sequentially passes between the following electrode positions: between FT8 and F8, between FC6 and F6, between FC6 and FC4, between C6 and C4, between CP6 and CP4, between P6 and P4, between P6 and PO6, and between P8 and PO8; the left lateral cranial region occupies at least 60%, or at least 70%, or at least 80% of the surface area of the third sub-region, and the right lateral cranial region occupies at least 60%, or at least 70%, or at least 80% of the surface area of the fourth sub-region.

5. The phototherapy device according to any one of claims 1-4, **characterized in that**, according to the 10-10 standard system,
the anterosuperior cranial region comprises electrode positions FP2, FPZ, FP1, AF3, AF4, AF7, AF8, AFZ, FZ, F1, F2, F3, F4, F5, F6, F7, F8, FC1, FC2, FC3, FC4, FCZ; and/or
the cranial vertex region comprises electrode positions CZ, C1, C2, C3, C4, CPZ, CP1, CP2, CP3, CP4, PZ, P1, P2, P3, P4; and/or
the left lateral cranial region comprises electrode positions FT7, FC5, T7, C5, TP7, CP5, P7, P5, and the right lateral cranial region comprises electrode positions FT8, FC6, T8, C6, TP8, CP6, P8, P6.

6. The phototherapy device according to any one of claims 1-4, **characterized in that**, according to the 10-10 standard system,
the anterosuperior cranial region comprises electrode positions AFZ, FZ, F1, F2, FP1, FP2; and/or
the cranial vertex region comprises electrode positions CZ, C1, C2, and CPZ; and/or
the left lateral cranial region comprises electrode positions FT7, FC5, T7, C5, or, TP7, CP5, P7 or P5, and the right lateral cranial region comprises electrode positions FT8, FC6, T8, C6, or, TP8, CP6, P8, P6.

7. The phototherapy device according to any one of claims 1-4, **characterized in that**, according to the 10-10 standard system,
the anterosuperior cranial region comprises electrode positions FP2, FPZ, FP1, AF3, AF4, AF7, AF8, AFZ, FZ, F1, F2, F3, F4, F5, F6, F7, FC5, F8, FC1, FC2, FC3, FC4, FCZ, FC6, CZ, C1, C2; and/or
the cranial vertex region comprises electrode positions C5, C3, C4, C6, CPZ, CP1, CP2, CP3, CP4, CP5, CP6, PZ, P1, P2, P3, P4; and/or
the left lateral cranial region comprises electrode positions FT7, T7, TP7, P7, and the right lateral cranial region comprises electrode positions FT8, T8, TP8, P8.

8. The phototherapy device according to any one of claims 1-4, **characterized in that**, according to the 10-10 standard system,
the anterosuperior cranial region comprises electrode positions FP2, FPZ, FP1, AF3, AF4, AF7, AF8, AFZ, FZ, F1, F2, F3, F4, F5, F6, FC1, FC2, FC3, FC4, FC5, FC6, FCZ, CZ, C1, C2; and/or
the cranial vertex region comprises electrode positions C3, C4, C5, C6, CPZ, CP1, CP2, CP3, CP4, CP5, CP6, PZ, P1, P2, P3, P4; and/or
the left lateral cranial region comprises electrode positions F7, FT7, T7, TP7, P5, and the right lateral cranial region comprises electrode positions F8, FT8, T8, TP8, P6.

9. The phototherapy device according to claim 3, **characterized in that** the irradiation spatial range further covers the posterior cranial region, wherein the posterior cranial region is located in a fifth sub-region divided from the total region by the fifth boundary line, and the fifth sub-region is located below the first region.

10. The phototherapy device according to any one of claims 1-4, **characterized in that** the irradiation spatial range further covers the posterior cranial region, wherein the posterior cranial region comprises electrode positions PO7, PO5, PO3, POZ, PO4, PO6, PO8, O1, OZ, and O2.

11. The phototherapy device according to any one of claims 1-4, **characterized in that** the irradiation spatial range further covers the posterior cranial region, wherein the posterior cranial region comprises at least the occipital region above the inion.

12. The phototherapy device according to any one of claims 1-4, **characterized in that** the spatiotemporally averaged irradiance over the cranial vertex region and the anterosuperior cranial region is at least 55 mW/cm², and the spatiotemporally averaged irradiance over the left lateral cranial region and the right lateral cranial region is at least 35 mW/cm².

13. The phototherapy device according to any one of claims 1-4, **characterized in that** the irradiation spatial range further covers the posterior cranial region, and the spatiotemporally averaged irradiance over the posterior cranial region is at least 13 mW/cm².

14. The phototherapy device according to any one of claims 1-4, **characterized in that** when the array of near-infrared irradiation units is distributed on a spatial curved surface covering the subject's head with a predetermined gap, the spatiotemporally averaged irradiance over the cranial vertex region and the anterosuperior cranial region is higher than the spatiotemporally averaged irradiance over the left lateral cranial region and the right lateral cranial region.

15. The phototherapy device according to any one of claims 1-4, **characterized in that** the irradiation spatial range further covers the posterior cranial region, and both the spatiotemporally averaged irradiance over the cranial vertex region and the anterosuperior cranial region, and the spatiotemporally averaged irradiance over the left lateral cranial region and the right lateral cranial region, are both higher than the spatiotemporally averaged irradiance over the posterior cranial region.

16. The phototherapy device according to any one of claims 1-4, **characterized in that** the spatiotemporally averaged irradiance over each region is determined based on the time-averaged irradiance at representative positions of each region, wherein the representative positions comprise at least one of the following:
electrode positions according to the 10-10 standard system contained within each region, or a predetermined proportion thereof;
electrode positions according to the 10-10 standard system contained within each region and non-electrode positions among the electrode positions, or a predetermined proportion thereof;
a predetermined number of positions selected in each region according to a predetermined manner, wherein the predetermined manner comprises at least one of the following: randomly selecting a predetermined number of positions; uniformly dividing each region into a predetermined number of grids and selecting the predetermined number of positions corresponding to the grids; selecting the predetermined number of positions excluding edge positions; selecting positions of projection points in each region for the centers of the near-infrared irradiation units.

17. The phototherapy device according to any one of claims 1-4, **characterized in that** the support mechanism comprises a hood disposed in the air, wherein the hood is shaped to form a cavity; when the subject's head is positioned in place in the accommodating space, the size of the cavity is suitable to accommodate the subject's head in a loose manner without applying pressure to the subject's head; each near-infrared irradiation unit in a first group of near-infrared irradiation units has a preset emission angle, and has a preset spacing and height difference configuration with other near-infrared irradiation units in the first group, such that multiple convergence regions are formed above the cranial vertex region and the anterosuperior cranial region, for converging near-infrared light at each convergence region.

18. The phototherapy device according to claim 17, **characterized in that**,
the corresponding near-infrared irradiation units above the cranial vertex region and the anterosuperior cranial region are arranged along an arched curved surface; and when the subject's head is positioned in place in the accommodating space, the corresponding near-infrared irradiation units are disposed at a first distance above the cranial vertex region and the anterosuperior cranial region, and the average of the first distance is greater than a predetermined average distance, such that the spatiotemporally averaged irradiance over the convergence regions above the cranial vertex region and the anterosuperior cranial region is at least 58 mW/cm².

19. The phototherapy device according to any one of claims 1-4, **characterized in that** the support mechanism comprises a hood disposed in the air, wherein the hood is shaped to form a cavity, and the hood supports at least three groups of near-infrared irradiation units at different heights, wherein each group is disposed at a corresponding height, and the near-infrared irradiation units within a group are circumferentially spaced at a common height.

20. The phototherapy device according to claim 19, **characterized in that** the cavity formed by the hood is bell-shaped; when the subject's head is positioned in place in the accommodating space, the hood is configured that its bilateral lower part align with the tragus or its periphery on both sides, its posterior edge is positioned within a predetermined distance from the inion, and its anterior edge extends above the brow ridge.

21. The phototherapy device according to claim 19, **characterized in that** the hood comprises a light-transmitting protective portion, wherein the light-transmitting protective portion forms an anterior upper part, a top part, a rear part, a left side part, and a right side part, and encloses the cavity;
the near-infrared light emergent from the top part, the anterior upper part, the rear part, the left side part, and the right side part of the light-transmitting protective portion forms an irradiation curved surface without non-irradiated regions on the surface of the calvarial region of the subject's head.

22. The phototherapy device according to claim 19, **characterized in that** the hood comprises a light-transmitting protective portion, wherein the light-transmitting protective portion forms an anterior upper part, a top part, a rear part, a left side part, and a right side part, and encloses the cavity; the light-transmitting protective portion is an integral housing, and near-infrared light is emergent from the inner walls at each positions of the light-transmitting protective portion.

23. The phototherapy device according to any one of claims 1-4, **characterized in that** each near-infrared irradiation unit comprises 4-9 near-infrared LEDs, wherein the time-averaged optical power of each near-infrared LED is 80 mW-100 mW, and the irradiation surface area of each near-infrared irradiation unit is 5-9 cm² at its emergent surface.

24. The phototherapy device according to any one of claims 1-4, **characterized in that** the phototherapy device further comprises a cooling mechanism, wherein the cooling mechanism is configured to: ensure that the temperature of the air in the space adjacent to but not contacting the subject's head does not exceed 41°C, under conditions where the time-averaged total radiant power incident upon the surface of the subject's head reaches 27.5-120 W and the duration of a single continuous irradiation reaches 30 minutes.

25. The phototherapy device according to any one of claims 1-4, **characterized in that** the array of near-infrared irradiation units is specifically configured to deliver irradiation energy of at least 8,260-42,260 Joules to the subject's head within a continuous irradiation time of 5 minutes, as a unit energy delivery.

26. The phototherapy device according to any one of claims 1-4, **characterized in that** the array of near-infrared irradiation units is specifically configured to deliver irradiation energy of at least 24,800 Joules-126,800 Joules to the subject's head within a single session of 1 hour using separated or continuous effective unit energy deliveries, as a single-session cumulative energy delivery.

27. The phototherapy device according to any one of claims 1-4, **characterized in that** the array of near-infrared irradiation units is specifically configured to deliver irradiation energy of at least 24,800 Joules-1,014,100 Joules to the subject's head within a single day, as a daily cumulative energy delivery.

28. The phototherapy device according to claim 27, **characterized in that** the array of near-infrared irradiation units is specifically configured to implement the single-session cumulative energy delivery by continuous irradiation for more than 30 minutes within a single session of 1 hour, and to implement up to 4 times within a single day, to provide an accelerated daily cumulative energy delivery.

29. The phototherapy device according to claim 26, **characterized in that** the array of near-infrared irradiation units is specifically configured to deliver the irradiation energy of at least 124,000 Joules-7,099,000 Joules to the subject's head within one week using separated single-session cumulative energy deliveries, as a weekly cumulative energy delivery, wherein within each day of the week during which irradiation is performed, the single-session cumulative energy delivery can be implemented in separate time slots for 1, 2, 3, 4, 5, 6, 7, or 8 times.

30. The phototherapy device according to claim 29, **characterized in that** the array of near-infrared irradiation units is specifically configured to implement more than 8 times the weekly cumulative energy delivery within 8 weeks, preferably more than 16 times the weekly cumulative energy delivery within 16 weeks, as a treatment course cumulative energy delivery.

31. The phototherapy device according to claim 30, **characterized in that** the treatment course interruption time between two treatment courses does not exceed half of the treatment course duration.
